# EUROPEAN PATENT APPLICATION

(11) **EP 3 868 402 A1**
(43) Date of publication of application: **25.08.2021**
(21) Application number: 19872827.1
(22) Date of filing: 11.10.2019
(51) Int. Cl.: A61K 39/395, A61K 38/17, A61K 38/39, A61K 45/00, A61P 19/02, A61P 29/00, A61P 43/00, G01N 33/15, G01N 33/50, G01N 33/53

(54) **MATURATION SUPPRESSOR AND MATURATION SUPPRESSION METHOD FOR DENDRITIC CELLS, AND PHARMACEUTICAL COMPOSITION**

(30) Priority: 15.10.2018 JP 2018194614
(71) Applicant: Tokyo University of Science Foundation, Tokyo 162-8601 (JP)
(72) Inventor: IWAKURA Yoichiro, Tokyo 162-8601 (JP); YABE Rikio, Tokyo 162-8601 (JP)
(74) Representative: Isarpatent
(86) International application number: PCT/JP2019/040246
(87) International publication number: WO 2020/080298

(57) **Abstract**

Provided are: a maturation suppressor for dendritic cells, said maturation suppressor inhibiting the binding of TARM1 protein to collagen protein to thereby suppress the maturation of dendritic cells; a maturation suppression method for dendritic cells, said method comprising using the maturation suppressor; a pharmaceutical composition containing the maturation suppressor as an active ingredient; a method for screening a maturation suppressor for dendritic cells; and a method for screening a therapeutic component for a disease mediated by dendritic cells.

## Description

### TECHNICAL FIELD

The present invention relates to a dendritic cell maturation-suppressing agent, a dendritic cell maturation-suppressing method, a pharmaceutical composition, and a method for screening a dendritic cell maturation-suppressing agent or a therapeutic ingredient for a dendritic cell-mediated disease.

### BACKGROUND ART

Antigen-presenting cells such as dendritic cells and macrophages express a diverse array of costimulatory receptors. The costimulatory receptors transmit activation signals to T cells cooperatively with interactions of MHC class I molecules and MHC class II molecules with T cell receptors to thereby activate acquired immune responses. Furthermore, the costimulatory receptors also transmit inhibitory signals for T cell anergy. These positive and negative signals are delicately controlled by interactions of receptors and ligands. Therefore, appropriate signaling by receptor-ligand axes of the antigen-presenting cells is extremely important for regulating immune response via T cells. For example, autoimmune diseases such as rheumatoid arthritis are closely related to a decrease in costimulation signals.

A leukocyte immunoglobulin-like receptor (LILR) family includes a number of immunoglobulin-like receptors. Some of the immunoglobulin-like receptors are expressed in myeloid cells such as dendritic cells and macrophages and mediate costimulation to immune cells. Some reports in the past suggested that genes in the LILR family are closely related to autoimmune diseases.

Recently, TARM1 (T cell-interacting, activating receptor on myeloid cells-1) was identified as an immunoglobulin-like receptor coded by a leukocyte-receptor complex region of the LILR family (see Non-Patent Document 1). The TARM1 is composed of two extracellular immunoglobulin-like domains, a transmembrane domain, and a short cytoplasmic domain having no typical signal motif and specifically associates with an FCRγ chain, an adapter molecule having ITAM (Immunoreceptor tyrosine-based activation motif). As a result of an amino acid sequence analysis, the TARM1 has been found to be highly homologous with human monocyte-derived dendritic cells or OSCAR (Osteoclast-associated receptor) mediating costimulation of mouse osteoclasts. The TARM1 promotes production of TNF-α and IL-6 from macrophages and neutrophils by cross-linking with anti-TARM1 monoclonal antibodies in the presence of Tolllike receptor (TLR) ligands. Furthermore, inflammatory stimuli such as LPSs (lipopolysaccharides) facilitate an expression of a TARM1 gene.

Patent Document 1 describes, for example, that dendritic cells express TARM (e.g., TARM1), that inflammatory stimuli facilitate an expression of the TARM, that the dendritic cells adhere to T cells via the TARM, that anti-TARM antibodies suppress the dendritic cells from adhering to the T cells, and that anti-TARM antibodies have a therapeutic effect for collagen-induced arthritis (CIA). However, a relationship between the TARM1 and maturation of the dendritic cells has still not be known.

Patent Document 1: PCT International Publication No. WO2007/037430

Non-Patent Document 1: V. Radjabova et al., J. Immunol., 195, 3149-3159, 2015

### DISCLOSURE OF THE INVENTION

### Problems to be Solved by the Invention

An object of the present invention is to provide a novel, dendritic cell maturation-suppressing agent capable of suppressing dendritic cell maturation; a dendritic cell maturation-suppressing method using such a maturation-suppressing agent; a pharmaceutical composition containing such a maturation-suppressing agent as an active ingredient; a method for screening a dendritic cell maturation-suppressing agent; and a method for screening a therapeutic ingredient for a dendritic cell-mediated disease.

### Means for Solving the Problems

Specific means for solving the above-mentioned object include the following embodiments.
<1> A dendritic cell maturation-suppressing agent being capable of inhibiting binding between a TARM1 protein and a collagen protein to suppress dendritic cell maturation.
<2> The dendritic cell maturation-suppressing agent according to <1>, the agent being capable of binding to a collagen-binding domain in the TARM1 protein or the collagen protein.
<3> The dendritic cell maturation-suppressing agent according to <2>, the agent being an antibody capable of binding to the collagen-binding domain in the TARM1 protein or the collagen protein, or a functional fragment of the antibody.
<4> The dendritic cell maturation-suppressing agent according to <2>, the agent being the TARM1 protein or the collagen protein, or a functional equivalent thereof.
<5> The dendritic cell maturation-suppressing agent according to <4>, the agent being any one of polypeptides (a) to (d) below or a fusion protein of the polypeptide with an IgG Fc region:
   (a) a polypeptide including at least amino acid residues at positions 27 to 120 or 124 to 219 from an N terminus in an amino acid sequence set forth in SEQ ID NO: 2;
   (b) a polypeptide including at least amino acid residues at positions 27 to 120 or 124 to 218 from an N terminus in an amino acid sequence set forth in SEQ ID NO: 4;
   (c) a polypeptide including an amino acid sequence in which one or several amino acid residues are substituted for, deleted from, or added to an amino acid sequence of the polypeptide (a) or (b) and having a binding capability for the collagen protein; and
   (d) a polypeptide including an amino acid sequence having 90% or more sequence identity to an amino acid sequence of the polypeptide (a) or (b) and having a binding capability for the collagen protein.
<6> The dendritic cell maturation-suppressing agent according to <4>, the agent being a collagen polypeptide, a polypeptide including a collagen-like domain, or a fusion protein of the collagen polypeptide or the polypeptide including a collagen-like domain with an IgG Fc region.
<7> A method for suppressing dendritic cell maturation, the method including:
   bringing the dendritic cell maturation-suppressing agent according to any one of <1> to <6> into contact with an immature dendritic cell.
<8> A pharmaceutical composition for use in treating a dendritic cell-mediated disease, including the dendritic cell maturation-suppressing agent according to any one of <1> to <6> as an active ingredient.
<9> The pharmaceutical composition according to <8> for use in treating rheumatoid arthritis.
<10> A screening method, including:
   measuring binding activity between any one of polypeptides (a) to (d) below and a collagen protein in the presence and
   absence of a test substance; and
   selecting the test substance as a candidate for a dendritic cell maturation-suppressing agent when the binding activity in the presence of the test substance is lower than the binding activity in the absence of the test substance:
      (a) a polypeptide including at least amino acid residues at positions 27 to 120 or 124 to 219 from an N terminus in an amino acid sequence set forth in SEQ ID NO: 2;
      (b) a polypeptide including at least amino acid residues at positions 27 to 120 or 124 to 218 from an N terminus in an amino acid sequence set forth in SEQ ID NO: 4;
      (c) a polypeptide including an amino acid sequence in which one or several amino acid residues are substituted for, deleted from, or added to an amino acid sequence of the polypeptide (a) or (b) and having a binding capability for the collagen protein; and
      (d) a polypeptide including an amino acid sequence having 90% or more sequence identity to an amino acid sequence of the polypeptide (a) or (b) and having a binding capability for the collagen protein.
<11> A screening method, including:
   measuring binding activity between any one of polypeptides (a) to (d) below and a collagen protein in the presence and absence of a test substance; and
   selecting the test substance as a candidate for a therapeutic ingredient for a dendritic cell-mediated disease when the binding activity in the presence of the test substance is lower than the binding activity in the absence of the test substance:
      (a) a polypeptide including at least amino acid residues at positions 27 to 120 or 124 to 219 from an N terminus in an amino acid sequence set forth in SEQ ID NO: 2;
      (b) a polypeptide including at least amino acid residues at positions 27 to 120 or 124 to 218 from an N terminus in an amino acid sequence set forth in SEQ ID NO: 4;
      (c) a polypeptide including an amino acid sequence in which one or several amino acid residues are substituted for, deleted from, or added to an amino acid sequence of the polypeptide (a) or (b) and having a binding capability for the collagen protein; and
      (d) a polypeptide including an amino acid sequence having 90% or more sequence identity to an amino acid sequence of the polypeptide (a) or (b) and having a binding capability for the collagen protein.

### Effects of the Invention

The present invention makes it possible to provide a novel, dendritic cell maturation-suppressing agent capable of suppressing dendritic cell maturation; a dendritic cell maturation-suppressing method using such a maturation-suppressing agent; a pharmaceutical composition containing such a maturation-suppressing agent as an active ingredient; a method for screening a dendritic cell maturation-suppressing agent; and a method for screening a therapeutic ingredient for a dendritic cell-mediated disease.

### BRIEF DESCRIPTION OF THE DRAWINGS

Fig. 1A illustrates a graph showing a result for quantitative PCR analysis of an expression of a Tarm1 gene in the joints of *I11rn*^{*-*/*-*} mice (*p < 0.05 (two-tailed unpaired Student's t-test)).
Fig. 1B illustrates a graph showing a result for quantitative PCR analysis of an expression of a Tarm1 gene in the joints of HTLV-I Tg mice (*p < 0.05 (two-tailed unpaired Student's t-test)).
Fig. 1C illustrates graphs showing results for quantitative PCR analysis of an expression of a Tarm1 gene and inflammatory cytokine genes in the joints of *I11rn*^{*-*/*-*} mice.
Fig. 1D illustrates graphs showing results for quantitative PCR analysis of an expression of a Tarm1 gene and inflammatory cytokine genes in the joints of HTLV-I Tg mice.
Fig. 1E illustrates a graph showing an incidence of arthritis when *Tarm1*^{egfp/egfp} mice and WT mice were immunized with a mixed emulsion of type II collagen and CFA (**p* < 0.05 (chi-square test).
Fig. 1F illustrates a graph showing a severity of arthritis when *Tarm1*^{egfp/egfp} mice and WT mice were immunized with a mixed emulsion of type II collagen and CFA (**p* < 0.05 (Mann-Whitney U-test).
Fig. 1G illustrates images showing joint conditions in *Tarm1*^{egfp/egfp} mice and WT mice on day 50 after immunization.
Fig. 1H illustrates images showing safranin O-stained arthritis tissues in *Tarm1*^{egfp/egfp} mice and WT mice on day 50 after immunization.
Fig. 1I illustrates images showing hematoxylin and eosin (HE)-stained arthritis tissues in *Tarm1*^{egfp/egfp} mice and WT mice on day 50 after immunization.
Fig. 1J illustrates graphs showing histopathological scores including synovitis, pannus formation, and bone erosion in *Tarm1*^{egfp/egfp} mice and WT mice on day 50 after immunization (*p < 0.05 (Mann-Whitney *U*-test).
Fig. 1K illustrates graphs showing results for flow cytometry analysis of immune cell (CD11c⁺, I-A/I-E⁺CD11c⁺, CD44⁺CD4⁺, CD19⁺) populations in *Tarm1*^{egfp/egfp} mice and WT mice on day 42 after immunization (**p* < 0.05; ***p* < 0.01 (two-tailed unpaired Student's t-test)).
Fig. 1L illustrates a graph showing a result for ELISA measurement of antibody titers of IgG1, IgG2a, IgG2b, and IgG3 specific for type II collagen in *Tarm1*^{egfp/egfp} mice and WT mice (***p* < 0.01 (two-tailed unpaired Student's t-test)).
Fig. 2A illustrates a graph showing a result for [³H]-thymidine uptake measurement after cells in the draining lymph nodes (dLNs) taken from *Tarm1*^{egfp/egfp} mice and WT mice on day 10 after CIA induction were re-stimulated with type II collagen (**p* < 0.05 (two-tailed unpaired Student's t-test)).
Fig. 2B illustrates a graph showing a result for ELISA measurement of amounts of TNF included in culture supernatants after cells in the draining lymph nodes were re-stimulated with type II collagens at various concentrations in vitro for 66 hours (**p* < 0.05 (two-tailed unpaired Student's t-test)).
Fig. 2C illustrates a graph showing a result for ELISA measurement of amounts of INF-γ included in culture supernatants after cells in the draining lymph nodes were re-stimulated with type II collagens at various concentrations in vitro for 66 hours (**p* < 0.05; ****p* < 0.001 (two-tailed unpaired Student's t-test)).
Fig. 2D illustrates a graph showing a result for ELISA measurement of amounts of IL-17 included in culture supernatants after cells in the draining lymph nodes were re-stimulated with type II collagens at various concentrations in vitro for 66 hours (****p* < 0.001 (two-tailed unpaired Student's t-test)).
Fig. 2E illustrates graphs showing results for flow cytometry analysis of an I-A/I-E⁺CD11c⁺ cell population in the draining lymph nodes in *Tarm1*^{egfp/egfp} mice and WT mice on day 10 after CIA induction (***p* < 0.01 (two-tailed unpaired Student's t-test)) .
Fig. 2F illustrates graphs showing results for flow cytometry analysis of an expression of I-A/I-E in CD11c⁺ cells in the draining lymph nodes in *Tarm1*^{egfp/egfp} mice and WT mice on day 10 after CIA induction (***p* < 0.001 (two-tailed unpaired Student's t-test)).
Fig. 2G illustrates a graph showing a result for T cell proliferation verified by [³H]-thymidine uptake measurement when dendritic cells and T cells were isolated from the draining lymph nodes in *Tarm1*^{egfp/egfp} mice and WT mice on day 10 after CIA induction and co-cultured in the presence of type II collagen (**p* < 0.05 (two-tailed unpaired Student's t-test)).
Fig. 3A illustrates a graph showing a result for quantitative RT-PCR analysis of an expression amount of a Tarm1 gene in GM-CSF-induced dendritic cells (GM-DCs), M-CSF-induced bone marrow-derived macrophages (BMMs), osteoclasts (OCs), T cells (Thy1.2⁺ cells), and B cells (B220⁺ cells).
Fig. 3B illustrates graphs showing results for flow cytometry analysis of an expression of a Tarm1 gene in conventional dendritic cells (cDCs; CD11b⁺CD11c⁺ and CD24⁺CD11c⁺) and plasmacytoid dendritic cells (pDCs; B220⁺CD11c⁺) in *Tarm1*^{egfp/+} mice and WT mice_{.}
Fig. 3C illustrates a graph showing results of flow cytometry analysis of an expression of a Tarm1 gene in inflammatory dendritic cells (iDCs; Ly6C⁺Ly6G⁻MHC II^{hi}CD11b⁺CD11c⁺) in *Tarm1*^{egfp/+} mice and WT mice on day 7 after CIA induction.
Fig. 3D illustrates graphs showing results for flow cytometry analysis of an expression of a Tarm1 gene in GM-DCs.
Fig. 3E illustrates graphs showing results for flow cytometry analysis of an expression of a Tarm1 gene in Flt3L-induced dendritic cells (FL-DCs).
Fig. 3F illustrates graphs showing results for quantitative RT-PCR analysis of an expression amount of a Tarm1 gene after GM-DCs were treated with inflammatory cytokines (TNF and IL-1β).
Fig. 3G illustrates graphs showing results for quantitative RT-PCR analysis of an expression amount of a Tarm1 gene after GM-DCs were treated with pathogen-associated molecular patterns (LPS, CpG, and poly(I:C)).
Fig. 3H illustrates a graph showing a result for flow cytometry analysis of percentages of CD11c⁺ cells on predetermined days after a GM-CSF treatment in *Tarm1*^{egfp/egfp} mouse-derived and WT mouse-derived GM-DCs.
Fig. 3I illustrates a graph showing a result of flow cytometry analysis of percentages of I-A/I-E^{hi}CD11c⁺ cells on predetermined days after a GM-CSF treatment in *Tarm1*^{egfp/egfp} mouse-derived and WT mouse-derived GM-DCs (**p* < 0.05; ***p <* 0.01; ****p* < 0.001 (two-tailed unpaired Student's t-test)).
Fig. 3J illustrates graphs showing results of flow cytometry analysis of percentages of dendritic cell activation markers (I-A/I-E, CD86, and CD80) on predetermined days after a GM-CSF treatment in *Tarm1*^{egfp/egfp} mouse-derived and WT mouse-derived GM-DCs (**p* < 0.05; ***p* < 0.01; ****p* < 0.001 (two-tailed unpaired Student's t-test)).
Fig. 3K illustrates a heat map of genes of which a difference in expression amount was more than 2 times between *Tarm1*^{egfp/egfp} mouse-derived and WT mouse-derived GM-DCs as analyzed for gene expression by DNA microarray.
Fig. 3L illustrates graphs showing results for flow cytometry measurement of a CFSE intensity on day 3 of coculture of OT-II Tg mouse-derived CFSE-labeled CD4⁺ T cells with *Tarm1*^{egfp/egfp} mouse-derived and WT mouse-derived GM-DCs in the presence of OVA at predetermined concentrations (***p* < 0.01; ****p* < 0.001 (two-tailed unpaired Student's t-test)).
Fig. 4A illustrates graphs showing results for flow cytometry analysis of a binding of *Tarm1*^{egfp/egfp} mouse-derived and WT mouse-derived GM-DCs to Tarm1-Fc.
Fig. 4B illustrates graphs showing results for flow cytometry analysis of a binding of trypsinized or untreated GM-DCs to Tarm1-Fc.
Fig. 4C illustrates graphs showing results for a binding property of Tarm1-Fc to type I collagen, type II collagen, or BSA immobilized on plates as verified by a solid phase-binding assay.
Fig. 4D illustrates a graph showing a result for a binding property of Tarm1-Fc to type II collagen immobilized on plates in the presence or absence of EDTA as verified by a solid phase-binding assay.
Fig. 4E illustrates graphs showing results for flow cytometry analysis of an expression of type I collagen and type II collagen on cell surfaces of GM-DCs.
Fig. 4F illustrates a graph showing a result for flow cytometry analysis of a binding of collagen-treated or untreated GM-DCs to Tarm1-Fc.
Fig. 4G illustrates images showing results for immunoblotting using an anti-type II collagen antibody of precipitates obtained from whole cell lysates (WCLs) of GM-DCs by immunoprecipitation using Tarm1-Fc or IgG Fc.
Fig. 4H illustrates graphs showing results of flow cytometry analysis of an expression of dendritic cell activation markers (CD86 and I-A/I-E) in *Tarm1*^{egfp/egfp} mouse-derived and WT mouse-derived GM-DCs stimulated with type II collagen (**p* < 0.05; ***p* < 0.01; ****p* < 0.001 (two-tailed unpaired Student's t-test)).
Fig. 4I illustrates graphs showing results for flow cytometry analysis using cytometric beads of cytokine concentrations (TNF, IL-6, and IL-10) in culture supernatants of *Tarm1*^{egfp/egfp} mouse-derived and WT mouse-derived GM-DCs stimulated with type II collagen (***p* < 0.01 (two-tailed unpaired Student's *t*-test)).
Fig. 5A illustrates a severity of arthritis when Tarm1-Fc and IgG Fc were administered to the joint cavities in the left and right ankle joints of DBA/1J mice in which CIA had been induced (**p* < 0.05; ***p* < 0.01 (Mann-Whitney U-test)).
Fig. 5B illustrates paw conditions when Tarm1-Fc and IgG Fc were administered to the joint cavities in the left and right ankle joints of DBA/1J mice in which CIA had been induced.
Fig. 5C illustrates images of hematoxylin and eosin (HE)-stained tissues in the ankle joints of DBA/1J mice that received Tarm1-Fc and IgG Fc.
Fig. 5D illustrates graphs showing histopathological scores including synovitis, pannus formation, and bone erosion in DBA/1J mice that received Tarm1-Fc and IgG Fc (**p* < 0.05 (Mann-Whitney U-test)).
Fig. 5E illustrates a graph showing a result for quantitative PCR analysis of an expression of cytokines (TNF, IL-6, IL-Iβ, IL-10, and IL-17a) genes in the joints of DBA/1J mice that received Tarm1-Fc and IgG Fc (**p* < 0.05 (two-tailed unpaired Student's t-test)).

### PREFERRED MODE FOR CARRYING OUT THE INVENTION

### <Dendritic cell maturation-suppressing agent >

A dendritic cell maturation-suppressing agent according to the present embodiment inhibits a binding of a TARM1 protein to a collagen protein to thereby suppress maturation of the dendritic cell.

A TARM1 protein is a protein expressed on a cell membrane of a dendritic cell and is known to exhibit enhanced expression upon inflammatory stimulation. Nucleotide sequence information on a gene coding for the TARM1 protein and amino acid sequence information on the TARM1 protein are available from the GenBank database at the National Center for Biotechnology Information (NCBI). By way of example, nucleotide and amino acid sequences of human TARM1 are shown in SEQ ID NOs: 1 and 2, and nucleotide and amino acid sequences of mouse TARM1 are shown in SEQ ID NOs: 3 and 4.

The present inventors have found that the TARM1 protein expressed on the cell membrane of the dendritic cell binds to a type II collagen protein also expressed on the cell membrane of the dendritic cell, in other words, the type II collagen protein is a ligand for the TARM1 protein; that the type II collagen protein regulates maturation of the dendritic cell in a TARM1-dependent manner; that the maturation of the dendritic cell is suppressed by inhibiting the binding of the TARM1 protein to the type II collagen protein using a fusion protein (TARM1-Fc) of an extracellular domain of the TARM1 protein with an IgG Fc region, and the like.

Furthermore, the present inventors also have found that the TARM1 protein binds to a type I collagen protein, in other words, the type I collagen is a ligand for the TARM1 protein. It is known that the maturation of the dendritic cell is promoted upon binding to the type I collagen protein (U. Brand et al., Eur. J. Immunol., 28, 1673-1680, 1998). Therefore, the maturation of the dendritic cell can be suppressed by inhibiting the binding of the TARM1 protein to the type I collagen protein.

The dendritic cell maturation-suppressing agent according to the present embodiment can suppress a dendritic cell-mediated immune response by suppressing the maturation of the dendritic cell. Therefore, the dendritic cell maturation-suppressing agent according to the present embodiment can be used as, for example, a therapeutic ingredient for a dendritic cell-mediated disease. In fact, the present inventors have confirmed that TARM1-Fc has a therapeutic effect in a collagen-induced arthritis (CIA) model. Note that, the collagen-induced arthritis (CIA) model is a model of rheumatoid arthritis which is one of autoimmune diseases.

The dendritic cell maturation-suppressing agent according to the present embodiment is not particularly limited as long as the agent has an effect of inhibiting the binding of the TARM1 protein to the collagen protein. Examples of the dendritic cell maturation-suppressing agent include a molecule inhibiting the binding of the TARM1 protein to the collagen protein by binding to a collagen-binding domain in the TARM1 protein, a molecule inhibiting the binding of the TARM1 protein to the collagen protein by binding to the collagen protein, and the like.

The dendritic cell maturation-suppressing agent according to the present embodiment may be an antibody binding to the collagen-binding domain in the TARM1 protein or the collagen protein, or a functional fragment thereof.

Examples of the antibody binding to the collagen-binding domain in the TARM1 protein include those recognizing a part or whole of the collagen-binding domain in the TARM1 protein as an epitope. As mentioned below, two immunoglobulin-like domains in the TARM1 protein have a binding capability for the collagen protein. Therefore, for example, those recognizing a part or whole of at least one immunoglobulin-like domain as an epitope can be used as the antibody binding to the TARM1 protein.

Examples of the antibody binding to the collagen protein include those recognizing at least part of the type I collagen protein or the type II collagen protein as an epitope.

An isotype of the antibody is not particularly limited and may be any of IgG, IgM, IgA, IgD, and IgE. Furthermore, the antibody may be a polyclonal antibody or a monoclonal antibody. Examples of the monoclonal antibody include a monoclonal antibody obtained via immunization of a non-human animal, a chimeric antibody, a humanized antibody, and a fully human antibody.

Examples of the functional fragment of the antibody include Fab, Fab', F(ab')₂, Fv, and scFv.

Furthermore, the dendritic cell maturation-suppressing agent according to the present embodiment may also be a small molecule compound, a polypeptide, a protein, a polysaccharide, and a nucleic acid, in addition to the antibodies.

As mentioned above, the TARM1 protein binds to the collagen protein. Therefore, the TARM1 protein or a functional equivalent thereof may also be used as the dendritic cell maturation-suppressing agent. Examples of the functional equivalent of the TARM1 protein include any of polypeptides (a) to (d) below or a fusion protein of the polypeptide with an IgG Fc region:
(a) a polypeptide including at least amino acid residues at positions 27 to 120 or 124 to 219 from an N terminus in an amino acid sequence set forth in SEQ ID NO: 2;
(b) a polypeptide including at least amino acid residues at positions 27 to 120 or 124 to 218 from an N terminus in an amino acid sequence set forth in SEQ ID NO: 4;
(c) a polypeptide including an amino acid sequence in which one or several amino acid residues are substituted for, deleted from, or added to an amino acid sequence of the polypeptide (a) or (b) and having a binding capability for the collagen protein; and
(d) a polypeptide including an amino acid sequence having 90% or more sequence identity to an amino acid sequence of the polypeptide (a) or (b) and having a binding capability for the collagen protein.

The polypeptide (a) includes at least amino acid residues at positions 27 to 120 or 124 to 219 from an N terminus in an amino acid sequence of a human TARM1 protein set forth in SEQ ID NO: 2. Regions at positions 27 to 120 and 124 to 219 from the N terminus correspond to two immunoglobulin-like domains in the human TARM1 protein. The polypeptide (a) including at least one immunoglobulin-like domain in the human TARM1 protein has the binding capability for the collagen protein.

Note that, it is known that an OSCAR protein highly homologous with the TARM1 protein also has two immunoglobulin-like domains and each of the immunoglobulin-like domains has the binding capability for the collagen protein (A. D. Barrow et al., J. Clin. Invest., 121, 3505-3516, 2011; L. Zhou et al., Blood, 127, 529-537, 2016; J. Haywood et al., Proc. Natl. Acad. Sci. USA, 113, 1038-1043, 2016).

The polypeptide (b) includes at least amino acid residues at positions 27 to 120 or 124 to 218 from an N terminus in an amino acid sequence of a mouse TARM1 protein set forth in SEQ ID NO: 4. Regions at positions 27 to 120 and 124 to 218 from the N terminus correspond to two immunoglobulin-like domains in the mouse TARM1 protein. The polypeptide (b) including at least one immunoglobulin domain in the mouse TARM1 protein has the binding capability for the collagen protein.

The polypeptide (c) includes an amino acid sequence in which one or several amino acid residues are substituted for, deleted from, or added to an amino acid sequence of the polypeptide (a) or (b) and has the binding capability for the collagen protein. The number of amino acid residues to be substituted for, deleted from, or added to is not particularly limited as long as the polypeptide retains the binding capability for the collagen protein. The number of amino acid residues to be substituted for, deleted from, or added to may be, for example, 1 to 30, 1 to 25, 1 to 20, or 1 to 15.

When one amino acid is substituted with another amino acid, usually, side chains of the amino acids preferably retain their property before and after the substitution. When amino acids are classified in accordance with properties of side chains of the amino acids, for example, the amino acids may be classified into hydrophilic amino acids (D, E, K, R, H, S, T, N, and Q); hydrophobic amino acids (A, G, V, I, L, F, Y, W, M, C, and P); acidic amino acids (D and E); basic amino acids (K, R, and H); amino acids having aliphatic side chains (A, G, V, I, and L); amino acids having aromatic-containing side chains (F, Y, and W); amino acids having sulfur atomcontaining side chains (M and C); and the like (alphabets within parentheses denotes one letter codes of amino acids).

The polypeptide (d) includes an amino acid sequence having 90% or more sequence identity to an amino acid sequence of the polypeptide (a) or (b) and has the binding capability for the collagen protein. The sequence identity to the polypeptide (a) or (b) may be 92% or more, 95% or more, 98% or more, or 99% or more.

Note that, the phrase "sequence identity" means consistency between sequences when two amino acid sequences are aligned and may be calculated using, for example, the BLAST program (www.ncbi.nlm.nih.gov/Blast/cgi).

Furthermore, as mentioned above, the collagen protein binds to the TARM1 protein. Therefore, the collagen protein or a functional equivalent thereof may be used as the dendritic cell maturation-suppressing agent. Examples of the functional equivalent of the collagen protein include a collagen polypeptide, a polypeptide including a collagen-like domain, and a fusion protein of the collagen polypeptide or the polypeptide including a collagen-like domain with an IgG Fc region.

### <Method for suppressing maturation of dendritic cell>

A method for suppressing maturation of a dendritic cell according to the present embodiment includes bringing the above-mentioned dendritic cell maturation-suppressing agent according to the present embodiment into contact with an immature dendritic cell. The dendritic cell maturation-suppressing agent is as mentioned above and a detailed description thereof is omitted.

A method for bringing the dendritic cell maturation-suppressing agent into contact with an immature dendritic cell is not particularly limited. Examples thereof include a method of culturing the immature dendritic cell in a medium containing the dendritic cell maturation-suppressing agent.

Note that, whether the dendritic cell is an immature dendritic cell or a mature dendritic cell may be determined by, for example, analyzing maturation markers of the dendritic cell such as CD40, CD80, CD83, CD86, and I-A/I-E with flow cytometry.

### <Pharmaceutical composition>

A pharmaceutical composition according to the present embodiment includes the above-mentioned dendritic cell maturation-suppressing agent according to the present embodiment as an active ingredient. The dendritic cell maturation-suppressing agent is as mentioned above and a detailed description thereof is omitted.

The pharmaceutical composition according to the present embodiment can suppress the maturation of the dendritic cell, and therefore, can be suitably used for treating a dendritic cell-mediated disease. In other words, the dendritic cell-mediated disease can be treated by administering the pharmaceutical composition according to the present embodiment to a patient with the disease. Examples of the dendritic cell-mediated disease include an autoimmune disease, an allergic disease, an inflammatory disease, and the like. Examples of the autoimmune disease include rheumatoid arthritis, systemic lupus erythematosus, Sjogren's syndrome, connective tissue disease, Guillain-Barre syndrome, myasthenia gravis, multiple sclerosis, Goodpasture's syndrome, Basedow's disease, and type I diabetes. Examples of the allergic disease include airway hyperreactivity (asthma), atopic dermatitis, and the like. Examples of the inflammatory disease include ulcerative colitis, Crohn's disease, psoriasis, psoriatic arthritis, nephritis, and the like.

Note that, the term "treating" includes suppression of acuteness of symptoms of the disease, in addition to elimination or alleviation of the symptoms.

The pharmaceutical composition according to the present embodiment may include ingredients other than the dendritic cell maturation-suppressing agent. For example, the pharmaceutical composition may include an organic or inorganic carrier conventionally used as formulation materials. This carrier is formulated as an excipient, a lubricant, a binder, a disintegrant, or the like in the case of a solid formulation; or a solvent, a solubilizing agent, a suspending agent, a tonicity adjusting agent, or a buffering agent, or the like in the case of a liquid formulation. Furthermore, a therapeutic agent for the autoimmune disease may include a formulation additive such as a preservative, an antioxidant, a surfactant, a pH adjusting agent, a coloring agent, a sweetener, or the like.

A dosage form of the pharmaceutical composition is not particularly limited. Examples of the dosage form of the pharmaceutical composition include an oral preparation such as a tablet, a capsule, a granule, a powder, a troche, a syrup, an emulsion, a suspension, a film, and the like; and a parenteral preparation such as an injection, an infusion, a topical agent, a suppository, a pellet, a transnasal agent, a transpulmonary agent (inhalant), an eye drop, and the like.

A dose of the pharmaceutical composition is appropriately determined depending on a subject (age, sex, body weight, etc.), an administration route, a target disease, a symptom, and the like. Usually, the dose of 1 to 5000 mg/day, preferably 5 to 2000 mg/day, more preferably 50 to 2000 mg/day per adult (body weight: 60 kg) may be administered in one or several divided doses. However, an actual dose may be widely varied based on a physician's discretion and may be outside of the above-mentioned range.

### <Screening method>

A first aspect of a screening method according to the present embodiment includes measuring a binding activity of any of polypeptides (a) to (d) below with a collagen protein in a presence and absence of a test substance; and selecting the test substance as a candidate for a dendritic cell maturation-suppressing agent when the binding activity in the presence of the test substance is lower than the binding activity in the absence of the test substance:
(a) a polypeptide including at least amino acid residues at positions 27 to 120 or 124 to 219 from an N terminus in an amino acid sequence set forth in SEQ ID NO: 2;
(b) a polypeptide including at least amino acid residues at positions 27 to 120 or 124 to 218 from an N terminus in an amino acid sequence set forth in SEQ ID NO: 4;
(c) a polypeptide including an amino acid sequence in which one or several amino acid residues are substituted for, deleted from, or added to an amino acid sequence of the polypeptide (a) or (b) and having a binding capability for the collagen protein; and
(d) a polypeptide including an amino acid sequence having 90% or more sequence identity to an amino acid sequence of the polypeptide (a) or (b) and having a binding capability for the collagen protein.

Furthermore, a second aspect of a screening method according to the present embodiment includes measuring a binding activity of any of the polypeptides (a) to (d) above with a collagen protein in a presence and absence of a test substance; and selecting the test substance as a candidate for a therapeutic ingredient for a dendritic cell-mediated disease when the binding activity in the presence of the test substance is lower than the binding activity in the absence of the test substance.

The polypeptides (a) to (d) are as mentioned above and a detailed description thereof is omitted.

The binding activity of any of the polypeptides (a) to (d) with the collagen protein may be measured according to known methods. For example, the binding activity may be measured by adding any of the polypeptides (a) to (d) to a plate on which the collagen protein is immobilized, leaving to stand for a predetermined time, removing an unbound polypeptide by, for example, washing, and measuring an amount of a bound polypeptide. When any of the polypeptides (a) to (d) is added, the polypeptide may be appropriately labeled with a label substance such as an enzyme, a radioisotope, a fluorescent dye, or the like.

The test substance is selected as a candidate for the dendritic cell maturation-suppressing agent or the therapeutic ingredient for a dendritic cell-mediated disease when the binding activity in the presence of the test substance is lower than the binding activity in the absence of the test substance. By way of example, the test substance may be selected as a candidate for the dendritic cell maturation-suppressing agent or the therapeutic ingredient for a dendritic cell-mediated disease when the binding activity in the presence of the test substance is 50% or lower relative to the binding activity in the absence of the test substance.

Note that, examples of the test substance include, but not limited to, a small molecule compound, a protein, a polypeptide, a polysaccharide, and a nucleic acid. The test substance may be a novel substance or a known substance.

### EXAMPLES

Hereinafter, the present invention will be more specifically described with reference to Examples, but the present invention is not limited to the following Examples.

### <Material and method>

### (Mouse)

CD57BL/6J mice, BALBc/A mice, and DBA/1J mice were purchased from Japan SLC, Inc. or CLEA Japan, Inc. Tarm1 heterozygous deficient mice (*Tarm1*^{egfp/+} mice) and Tarm1 homozygous deficient mice (*Tarm1*^{egfp/egfp} mice) were generated by homologous recombination of an exon 1 of a Tarm1 gene in the C57BL/6J mice with an eGFP gene and a neomycin-resistant gene. The *Tarm1*^{egfp/egfp} mice were born at an expected Mendelian ratio and exhibited no clear abnormality. OT-II TCR transgenic mice (OT-II Tg mouse) were kindly provided by Dr. H. Kishimoto, the Research Institute for Biomedical Sciences, the Tokyo University of Science.

All mice were bred under SPF conditions in a clean room at the Research Institute for Biomedical Sciences, the Tokyo University of Science. For experiments, age (6- to 12-week-old)- and sex-matched mice were used. Animal experiments were approved by Committee at the Tokyo University of Science and conducted in accordance with the ethical guideline for animal experimentation and the guideline for safety of genetic engineering techniques.

### (Collagen-induced arthritis (CIA))

CIA was induced as follows according to the previous report (D. D. Brand et al., Nature protocols, 2, 1269-1275, 2007; J. J. Inglis et al., Nature protocols, 3, 612-618, 2008). Complete Freund's adjuvant (CFA; 1 mg/mL) was prepared by adding heat-killed M. tuberculosis (H37Ra; Difco) to Incomplete Freund's adjuvant (IFA; Thermo Fisher Scientific). An emulsion was prepared by mixing 4 mg/mL of type II collagen (SIGMA-ALDRICH) with CFA at a volume ratio of 1:1. A total of 200 µL of the emulsion was intradermally administered to 3 positions near the base of the tail of each of the mice (8- to 10-week-old). Fourteen days after the priming, the same volume of the emulsion was administered as a booster. Swelling of each paw was scored and evaluated as follows: 0: No change, 1: Swelling or focal redness of the finger joint, 2: Mild swelling of the ankle joint, 3: Severe swelling of the entire paw. Scores of four paws were added, so that the maximum score was 12.

For verification of a therapeutic effect of Tarm1-Fc for autoimmune arthritis, Tarm1-Fc (1 µg per ankle joint) or IgG Fc (1 µg per ankle joint) were administered to the joint cavities of the left or right ankle joints of the DBA/1J mice in which CIA (mild score) had been induced. On day 0, male DBA1/J mice (6- to 8-week-old) were immunized with 100 µL of emulsion composed of 2 mg/mL type II collagen and 1.65 mg/mL CFA (M. A. Murayama et al., Nature communications, 6, 8483, 2015). On day 21, the same volume of the emulsion was further administered as a booster. Progression of arthritis was scored and evaluated as follows: 0: No change, 1: Erythema and mild swelling of the tarsal joint, 2: Erythema and mild swelling extending from the tarsal joint to the digit, 3: Erythema and moderate swelling extending from the metatarsal joint, 4: Erythema and severe swelling extending to the ankle, the paw, and the digit, or ankylosis of the paw. Scores of four paws were added, so that the maximum score was 16.

### (Histopathology)

The mice were sacrificed under anesthesia followed by amputating the paws. The paws were fixed in 10% neutral buffered formalin and decalcified in 10% EDTA. Preparation of paraffin-embedded sections and immunohistochemical staining were delegated to the Institute of Medical Science, the University of Tokyo. The procedures will be briefly described. Serial tissue sections (5 µm) were stained with hematoxylin and eosin (HE) and safranin O, and scored and evaluated as follows (J. J. Inglis et al., Nature protocols, 3, 612-618, 2008): 0: No inflammatory sign, 1: Mild inflammation with synovial surface hyperplasia but without cartilage erosion, 2: Change of Grade 1 and granulomatous lesion in the synovial sublining, 3: Change of Grade 2, and pannus formation and cartilage/bone erosion. Indexes of arthritis in the ankle joints were estimated from grades of the bones including the talus, the tibia, and the calcaneus.

### (Antibody titer)

Chicken type II collagen-specific IgG was measured for antibody titer by ELISA. Sera were collected on day 42 after immunization. 96-well plates were coated with chicken type II collagen (20 µg/mL), added with serially diluted sera, and incubated at room temperature for 1 hour. The plates were washed with PBS/0.05% Tween 20, added with an alkaline phosphatase-labeled rabbit anti-mouse IgG antibody (Zymed), and incubated at room temperature for 1 hour. Then, SIGMA 104 phosphatase substrate (SIGMA-ALDRICH) was added thereto and absorbance at a wavelength of 415 nm was measured with a microplate reader (MTP-300; Hitachi).

### (Flow cytometry)

Flow cytometry was performed according to the previous report (R. Yabe et al., FEBS Journal, 277, pp. 4010-4026, 2010). The procedures will be briefly described. Cells were blocked with a 2.4G2 antibody and then stained by treating with a fluorescence-labeled antibody at 4°C for 30 minutes. Then, the cells were analyzed with a flow cytometer (FACS Canto II or FACSVerse; Becton Dickinson) and the resultant data were analyzed with FlowJo (Tree Star). Dead cells were stained with 7AAD (SIGMA-ALDRICH) and PI (SIGMA-ALDRICH).

For staining a dendritic cell subset, the lymph nodes were subjected to enzymolysis with 200 U/mL of type VIII collagenase (SIGMA-ALDRICH).

For cell sorting, a single cell suspension was incubated with a biotinylated antibody and then with anti-biotin beads (Miltenyi Biotec). The thus-labeled cells were isolated with a magnetic cell sorting system (autoMACS; Miltenyi Biotec) in a negative selection mode. Negative cells were stained with a fluorescence-labeled antibody, and CD11c⁺ cells and CD4⁺CD3⁺ cells were highly purified with a flow cytometer (FACS Aria II (Becton Dickinson) or MoFlo XDP (Beckman Coulter)).

For screening of a Tarm1 ligand, cells were blocked with a 2.4G2 antibody and then incubated with Tarm1-Fc on ice for 1 hour. Then, the cells were washed and further incubated with a PE-labeled anti-human IgG-Fc antibody (HP6017; BioLegend).

Antibodies used for the cytometry analysis are presented in Table 1.

**[Table 1]**

| Antibody name | Clone name | manufacturer |
|---|---|---|
| APC-conjugated anti-CD3e | 145-2C11 | Biolegend |
| APC/Cy7-conjugated anti-CD3e | 145-2C11 | Biolegend |
| Pacific blue -conjugated anti-CD3e | 145-2C11 | Biolegend |
| FITC-conjugated anti-CD4 | RM4-5 | Biolegend |
| PE-conjugated anti-CD4 | RM4-5 | Biolegend |
| APC-conjugated anti-CD4 | RM4-5 | Biolegend |
| PE/Cy7-conjugated anti-CD4 | RM4-5 | Biolegend |
| Pacific blue-conjugated anti-CD4 | RM4-5 | Biolegend |
| PE-conjugated anti-CD8 | 53-6.7 | Biolegend |
| APC/Cy7-conjugagted anti-CD8 | 53-6.7 | Biolegend |
| PE/Cy7-conjugated anti-CDllb | M1/70 | Biolegend |
| Pacific blue -conjugated anti-CDllb | M1/70 | Biolegend |
| PE-conjugated anti-CDllc | N418 | Biolegend |
| APC-conjugated anti-CDllc | N418 | Biolegend |
| Brilliant Violet 421-conjugated anti-CDllc | N418 | Biolegend |
| APC-conjugated anti-CD19 | 6D5 | Biolegend |
| PE/Cy7-conjugated anti-CD24 | 53-6.7 | Biolegend |
| PE-conjugated anti-CD44 | IM7 | Biolegend |
| PE-conjugated anti-B220 | RA3-6B2 | Biolegend |
| Biotin-conjugated anti-B220 | RA3-6B2 | Biolegend |
| PE-conjugated anti-CD80 | 16-10A1 | Biolegend |
| APC-conjugated anti-CD86 | GL1 | Biolegend |
| APC/Cy7-conjugagted anti-Ly6C | HK1.4 | Biolegend |
| Pacific blue-conjugated anti-Ly6G | 1A8 | Biolegend |
| APC/Cy7-conjugagted anti-I-A/I-E | M5/114.15.2 | Biolegend |
| Biotin-conjugated anti-Ter119 | TER-119 | BD pharmingen |

### (Proliferative recall assay)

The inguinal lymph nodes were isolated from mice sacrificed on day 10 after priming. Single-cell suspensions were prepared from the draining lymph nodes and cultured in 96-well plates in the presence of heat-denatured chicken type II collagen (2 x 10⁵/well) at predetermined concentrations. After incubation for 66 hours, the cells were labeled by treating with [³H] thymidine (0.25 µCi/mL; PerkinElmer) for 6 hours. The cells were harvested with Micro 96 cell harvester (Skatron) and [³H] radioactivity was measured with Micro Beta Systems (Pharmacia Biotech). Culture supernatants were collected 66 hours after stimulation.

### (Measurement of cytokine production)

Production of cytokines was measured using Mouse TNF ELISA MAX (BioLegend), Duo set IFN-γ (R&D systems), Duo set IL-17 (R&D systems), Cytometric Bead Array (CBA) mouse TNF Flex Set (BD Biosciences), CBA mouse IL-6 Flex Set (BD Biosciences), and CBA mouse IL-10 Flex Set (BD Biosciences).

### (Reverse transcription and quantitative-PCR)

RNA was extracted from tissues and cells and purified with Sepasol-RNA I Super (Nacalai Tesque) and GenElute mammalian total RNA miniprep kit (SIGMA-ALDRICH). The resultant RNA was reverse-transcribed with high capacity cDNA reverse transcription kit (Applied Biosystems). For quantitative-PCR, SYBR Premix Ex Taq I kit (TaKaRa) or SYBRP Premix Ex Taq II kit (TaKaRa) was used along with primer sets presented in Table 2 below.

**[Table 2]**

| Gene name | Primer sequence | SEQ ID NO |
|---|---|---|
| *Tarm1* | Forward 5'-TCTCTAGGCTCCTTTCCCTT-3' | 5 |
| | Reverse 5'-GTCACGTGGCTCTTGGT-3' | 6 |
| *Gapdh* | Forward 5'-TTCACCACCATGGAGAAGGC-3' | 7 |
| | Reverse 5'-GGCATGGACTGTGGTCATGA-3' | 8 |
| *Tnf* | Forward 5'-GCCTCCCTCTCATCAGTTCT-3' | 9 |
| | Reverse 5'-CACTTGGTGGTTTGCTACGA-3' | 10 |
| *I1 6* | Forward 5'-GAGGATACCACTCCCAACAGACC-3' | 11 |
| | Reverse 5'-AAGTGCATCATCGTTGTTCATACA-3' | 12 |
| *I11b* | Forward 5'-CAACCAACAAGTGATATTCTCCATG-3' | 13 |
| | Reverse 5'-GATCCACACTCTCCAGCTGCA-3' | 14 |
| *I110* | Forward 5'-GTGGAGCAGGTGAAGAGTGATTT-3' | 15 |
| | Reverse 5'-TCCCTGGATCAGATTTAGAGAGC-3' | 16 |

### (Preparation of bone marrow-derived dendritic cell)

Bone marrow was extracted from the tibia and the femur of C57BL/6J mice and bone marrow cells were collected by erythrocyte destruction with a hemolysis buffer (140 mM NH₄Cl, 17 mM Tris-HCl, pH 7.2).

For preparation of GM-CSF-induced dendritic cells (GM-DCs), the bone marrow cells were cultured in an RPMI 1640 medium containing 10% FBS, 100 U/mL penicillin, 100 µg/mL streptomycin, 0.1% 2-mercaptoethanol, and recombinant mouse GM-CSF (2 to 20 ng/mL; Peprotech) at a cell density of 2 x 10⁵/mL. On day 3 of culture, the same volume of the medium was further added to wells. On days 6 and 8 of culture, lowadherent cells were collected and cultured in a fresh medium containing GM-CSF (1 to 10 ng/mL). Non-adherent cells were used as GM-DCs.

For preparation of Flt3L-induced dendritic cells (FL-DCs), the bone marrow cells were cultured in an RPMI 1640 medium containing 10% FBS, 100 U/mL penicillin, 100 µg/mL streptomycin, 0.1% 2-mercaptoethanol, and recombinant mouse Flt3L (100 ng/mL; Peprotech) at a cell density of 2 x 10⁶/mL. Adherent cells were collected by pipetting with 2.5 mM EDTA/PBS and used as FL-DCs.

For preparation of M-CSF-induced bone marrow-derived macrophages (BMMs), the bone marrow cells were cultured in an RPMI 1640 medium containing 10% FBS, 100 U/mL penicillin, 100 µg/mL streptomycin, 0.1% 2-mercaptoethanol, and recombinant human M-CSF (20 ng/mL; R&D systems) at a cell density of 2 x 10⁶/mL. On day 3 of culture, a half volume of the medium was further added to wells. Adherent cells were collected by detaching the cells with 2.5 mM EDTA/PBS and used as BMMs.

### (Stimulation to GM-DC)

GM-DCs were cultured at 37°C for predetermined periods of time in the presence of LPS (100 ng/mL; SIGMA-ALDRICH) of E. coli 0111:B4, CpG (1 µM; Eurofins Genomics), poly(I:C) (100 µg/mL; InvivoGen), TNF (10 ng/mL; Peprotech), IL-1β (10 ng/mL; Peprotech), or type II collagen (1 µg/mL, 10 µg/mL, or 100 µg/mL; SIGMA-ALDRICH).

### (DNA microarray)

DNA microarray analysis was delegated to The Chemicals Evaluation and Research Institute, Japan. The procedures will be briefly described. The extent of degradation and quality of RNA were checked with 2100 Bioanalyzer (Agilent Technologies). The RNA was reverse-transcribed to cDNA and then transcribed to Cy3-labeled cRNA, a transcript, using Low Input Quick Amp Labeling Kit (Agilent). The resultant sample was hybridized to SurePrint G3 Mouse GE Microarray (Ver.2.0) in an oven and the microarray was scanned with DNA Microarray Scanner (Agilent Technologies).

Genes of which expression was down-regulated to half or less in GM-DCs of *Tarm1*^{egfp/egfp} mice were subjected to a gene ontology analysis with PANTHER Overrepresentation Test and modified with Bonferroni correction.

### (Coculture of GM-DC and T cell)

Cells isolated from the spleens and the lymph nodes of OT-II Tg mice were blocked with a 2.4G2 antibody, treated with a biotinylated anti-CDllc antibody (BioLegend), a biotinylated anti-CDllb antibody (BioLegend), or a biotinylated anti-B220 antibody (BioLegend), and treated with anti-biotin beads (Miltenyi Biotec). The thus-labeled cells were removed with a magnetic cell sorting system (autoMACS; Miltenyi Biotec). A negative fraction was further treated with anti-CD4 beads (Miltenyi Biotec) and the thus-labeled cells (CD4⁺ T cells) were collected with a magnetic cell sorting system. The CD4⁺ T cells were incubated with 5 µM CFSE (Invitrogen) at 37°C for 5 minutes. Then, the thus-CFSE-labelled CD4⁺ T cells (2 x 10⁵/well) were co-cultured with GM-DCs (2 x 10⁴/well) at 37°C for 3 days in the presence of OVA (1 µg/mL, 10 µg/mL, or 100 µg/mL). Cell proliferation was analyzed with flow cytometry.

### (Fc fusion protein)

IgG Fc fusion proteins were synthesized according to the previous report (R. Yabe et al., FEBS Journal, 277, 4010-4026, 2010). Lipofectamine LTX (Invitrogen) was used to transfect 293T cells with plasmids. Twenty-four hours after the transfection, the medium was exchanged with an OPTI-MEM medium (Invitrogen) containing 2% low-IgG FBS (Invitrogen). Then, fusion proteins secreted into the medium were purified by affinity chromatography with a Protein A Sepharose carrier.

### (Tryptic digestion and collagenase digestion)

Cells were treated with a trypsin-EDTA solution (0.05% or 0.02%; SIGMA-ALDRICH) or 200 U/mL collagenase (Wako chemicals) at 37°C for 30 minutes.

### (Solid phase binding assay)

Maxisorp 96-well plates (ThermoFisher SCIENTIFIC) were coated with type I collagen, type II collagen, or BSA (0 to 10 µg/mL), washed with a washing buffer (TBS containing 0.05% Tween 20 and 2 mM CaCl₂) , and then blocked with a blocking buffer (TBS containing 0.05% Tween 20 and 5% BSA). A binding buffer (TBS containing 0.05% Tween 20, 2mM CaCl₂, and 0.5% BSA) containing the IgG Fc fusion protein (5 µg/mL) was incubated at room temperature for 2 hours. An HRP-labeled goat anti-human IgG antibody (Fcγ fragment-specific; 5000-fold diluted; Jackson ImmunoResearch) was incubated at room temperature for 1 hour. Then, SureBlue TMB Microwell Peroxidase Substrate (SeraCare) was added as a color development reagent to each well and the reaction was quenched with 1N HCl. Color development was monitored at a wavelength of 450 nm using iMark microplate reader (BIO-RAD).

### (Immunoprecipitation with Tarm1-Fc and western blotting)

Cells were lysed with TBS containing 1% NP-40 and a protease inhibitor cocktail (for mammal cell/tissue extracts) (Nacalai tesque). The resultant cell lysate was pre-cleared and then incubated at 4°C overnight with a Protein A Sepharose carrier on which Tarm1-Fc or IgG Fc had been immobilized. After washing, the resultant slurry was heated at 95°C for 5 minutes with an SDS sample buffer. The thus-heated sample was centrifuged, the supernatant thereof was subjected to SDS-PAGE and then electroblotting onto a PVDF membrane. Then, the PVDF membrane was blocked with 4% Block Ace (DS Pharma Biomedical Co.), incubated with biotinylated anti-type II collagen antibodies (A2-10, F10-21, D8-6, and D1-2G; Chondrex), and further incubated with HRP-labeled streptavidin (BioLegend). Thereafter, the PVDF membrane was incubated with ECL Prime Western Blotting Detection System (GE Healthcare) and chemiluminescence was detected with a digital imager (LAS 4000 system; Fuji Film Life Science).

### (Statistical processing)

For statistical processing, the two-tailed Student's t-test, the chi-square test, and the Mann-Whitney U-test were used and a p-value less than 0.05 was determined as statistically significant (**p* < 0.05; ***p* < 0.01; ****p* < 0.001). Data were presented as mean ± SD.

### <Test Example 1>

It has been conventionally suggested by a gene expression analysis that an immunoglobulin-like gene in a leukocyte immunoglobulin-like receptor (LILR) family is involved in progression of arthritis (N. Fujikado et al., Arthritis research & therapy, 8, R100, 2006). Therefore, firstly, expression of a Tarm1 gene in the ankles of IL-1 receptor antagonist gene (*I11rn*) deficient mice (*I11rn*^{*-*/*-*} mice) and human T-cell leukemia virus type I (HTLV-I) transgenic mice (HTLV-I Tg mice) (R. Horai et al., The Journal of experimental medicine, 191, 313-320, 2000; Y. Iwakura et al., Science, 253, 1026-1028, 1991) was verified. Both the *I11rn*^{*-*/*-*} mice and the HTLV-I Tg mice spontaneously develop arthritis similar to rheumatoid arthritis.

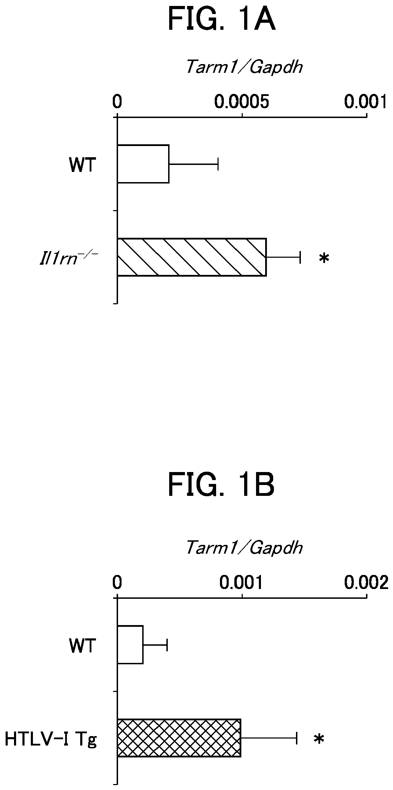

Figs. 1A and 1B show results for quantitative PCR analysis of the expression of the Tarm1 gene in the joints of the *I11rn*^{*-*/-} mice and the HTLV-I Tg mice (*I11rn*^{-/-} mice; n = 3, HTLV-I Tg mice; n = 4). The expression of the Tarm1 gene in WT mice was also analyzed as a control (n = 4). As shown in Figs. 1A and 1B, the expression of the Tarm1 gene was significantly enhanced in the *I11rn*^{*-*/*-*} mice and the HTLV-I Tg mice which developed arthritis compared to the WT mice.

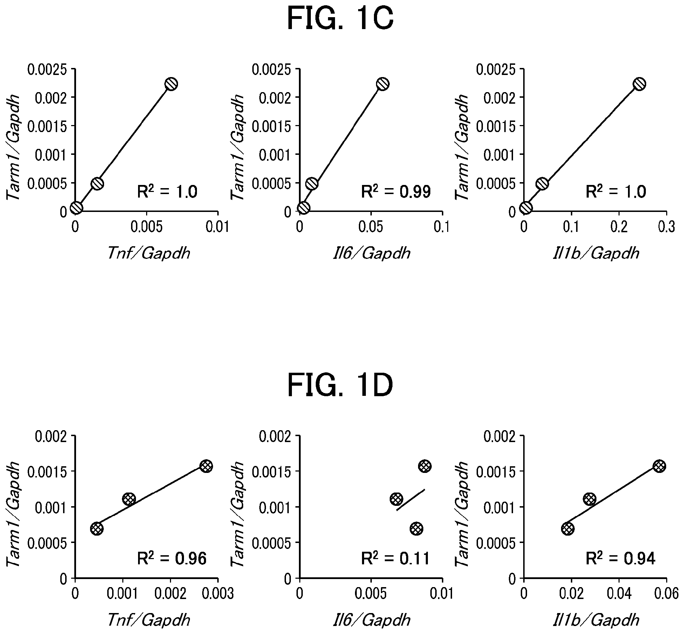

Then, a correlation between the expression of the Tarm1 gene and expression of inflammatory cytokine (TNF, IL-6, and IL-1β) genes was examined. Figs. 1C and 1D show results for quantitative PCR analysis of the expression of the Tarm1 gene and the inflammatory cytokine genes in the joints of the *I11rn*^{*-*/-} mice and the HTLV-I Tg mice (*I11rn*^{*-*/*-*} mice; n = 3, HTLV-I Tg mice; n = 3). The expression of the Tarm1 gene and the inflammatory cytokine genes in the WT mice was also analyzed as a control (n = 3). As shown in Figs. 1C and 1D, the expression of the Tarm1 gene was well correlated with the expression of the inflammatory cytokine genes.

Then, Tarm1 homozygous deficient mice (*Tarm1*^{egfp/egfp} mice) were examined for sensitivity against collagen-induced arthritis (CIA).

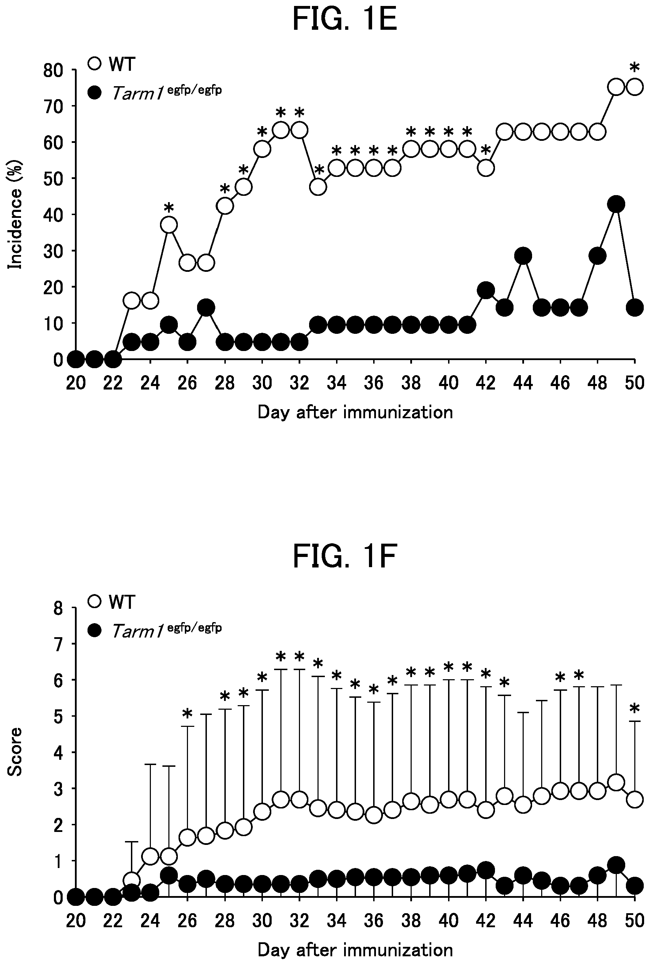

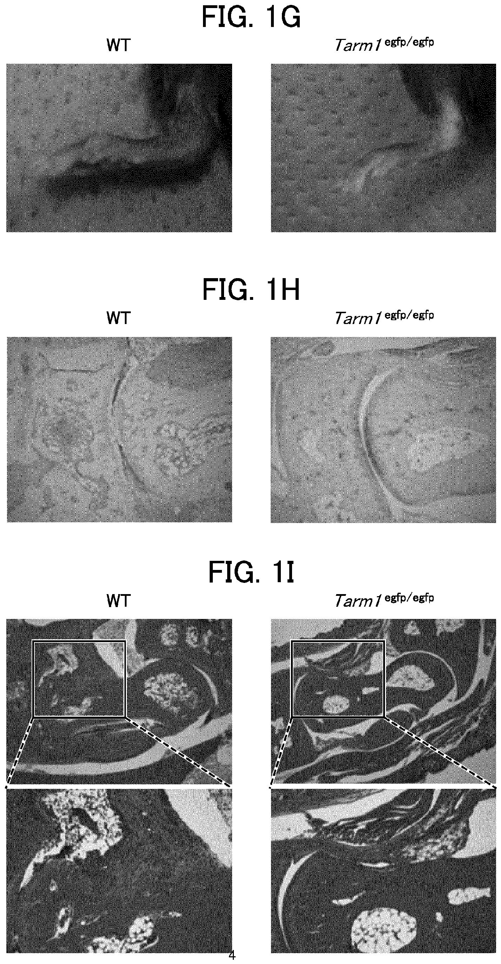

Figs. 1E and 1F show an incidence and a severity of arthritis when the *Tarm1*^{egfp/egfp} mice and the WT mice were immunized with a mixed emulsion of type II collagen and complete Freund's adjuvant (CFA) (*Tarm1*^{egfp/egfp} mice; n = 22, WT mice; n = 19). Figs. 1E and 1F show a combination of two independent experiments. In Fig. 1F, the sum of scores was divided by the total number of mice. Furthermore, Fig. 1G shows joint conditions (representative examples) in the *Tarm1*^{egfp/egfp} mice and the WT mice on day 50 after immunization. As shown in Figs. 1E to 1G, the incidence and the severity of arthritis were remarkably decreased in the *Tarm1*^{egfp/egfp} mice compared to the WT mice.

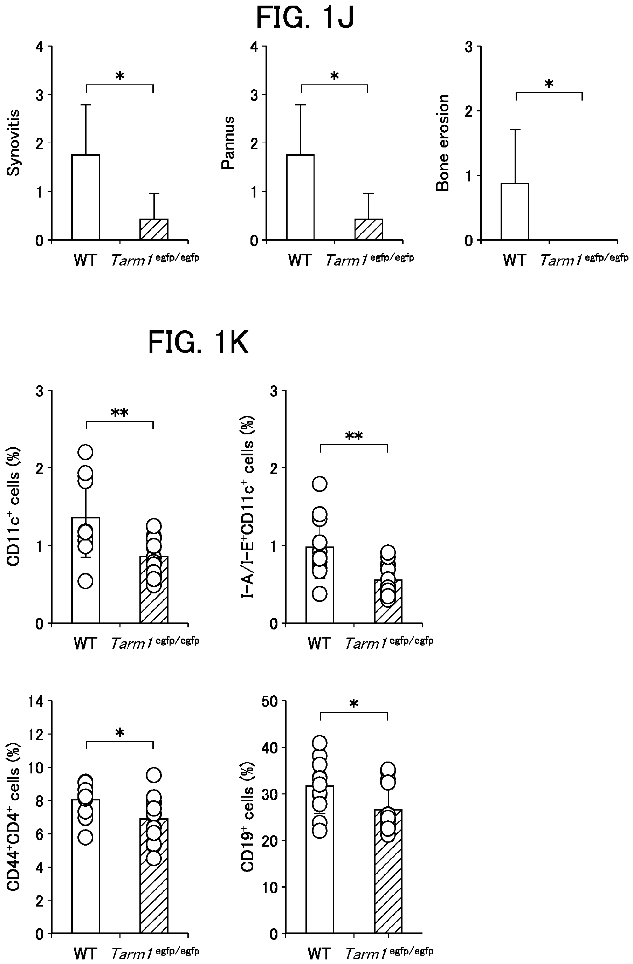

Figs. 1H and 1I show tissue images of arthritis regions (representative examples) in the *Tarm1*^{egfp/egfp} mice and the WT mice on day 50 after immunization. Fig. 1H shows sections (5 µm) of hind paws stained with safranin O and Fig. 1I shows sections (5 µm) of hind paws stained with hematoxylin and eosin (HE). Furthermore, Fig. 1J shows histopathological scores including synovitis, pannus formation, and bone erosion (*Tarm1*^{egfp/egfp} mice; n = 7, WT mice; n = 8). As shown in Figs. 1H to 1J, the synovitis, the pannus formation, and the bone erosion were remarkably decreased in the *Tarm1*^{egfp/egfp} mice compared to the WT mice.

Then, immune cell (CD11c⁺, I-A/I-E⁺CD11c⁺, CD44⁺CD4⁺, and CD19⁺) populations in the *Tarm1*^{egfp/egfp} mice and the WT mice on day 42 after immunization were analyzed by flow cytometry. Fig. 1K shows immune cell populations in the inguinal lymph nodes of the *Tarm1*^{egfp/egfp} mice and the WT mice (*Tarm1*^{egfp/egfp} mice; n = 14, WT mice; n = 11). As shown in Fig. 1K, the immune cell populations in the inguinal lymph nodes were significantly decreased in the *Tarm1*^{egfp/egfp} mice compared to the WT mice. This result is consistent with suppression of inflammation and bone erosion.

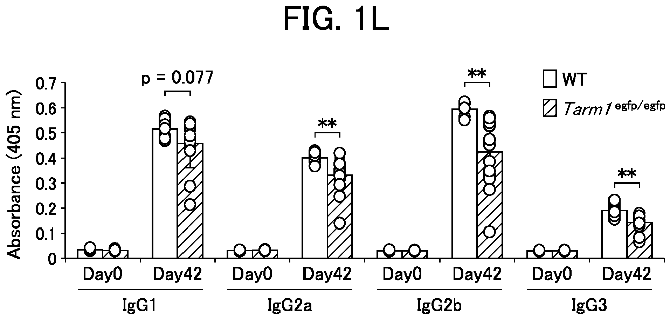

Then, antibody titers of serum IgGs specific for type II collagen in the *Tarm1*^{egfp/egfp} mice and the WT mice were measured with ELISA. Fig. 1L shows the antibody titers of IgG1, IgG2a, IgG2b, and IgG3 specific for the type II collagen (*arm1*^{egfp/egfp} mice; n = 14, WT mice; n = 11). As shown in Fig. 1L, the antibody titers of IgG2a, IgG2b, and IgG3 were significantly decreased in the *Tarm1*^{egfp/egfp} mice compared to the WT mice.

It was suggested from the above results that the *Tarm1*^{egfp/egfp} mice were more resistant to CIA compared to the WT mice.

### <Test Example 2>

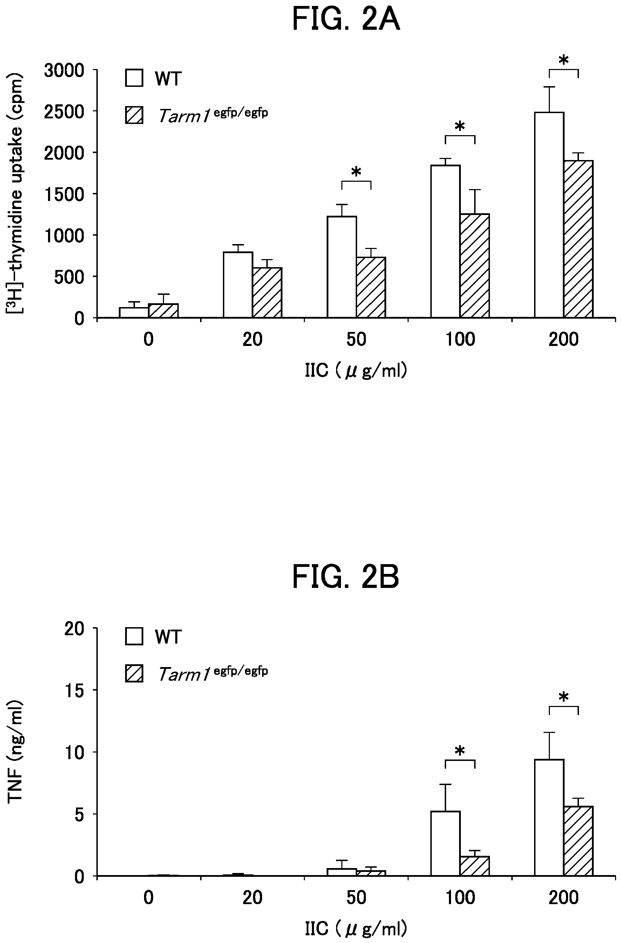

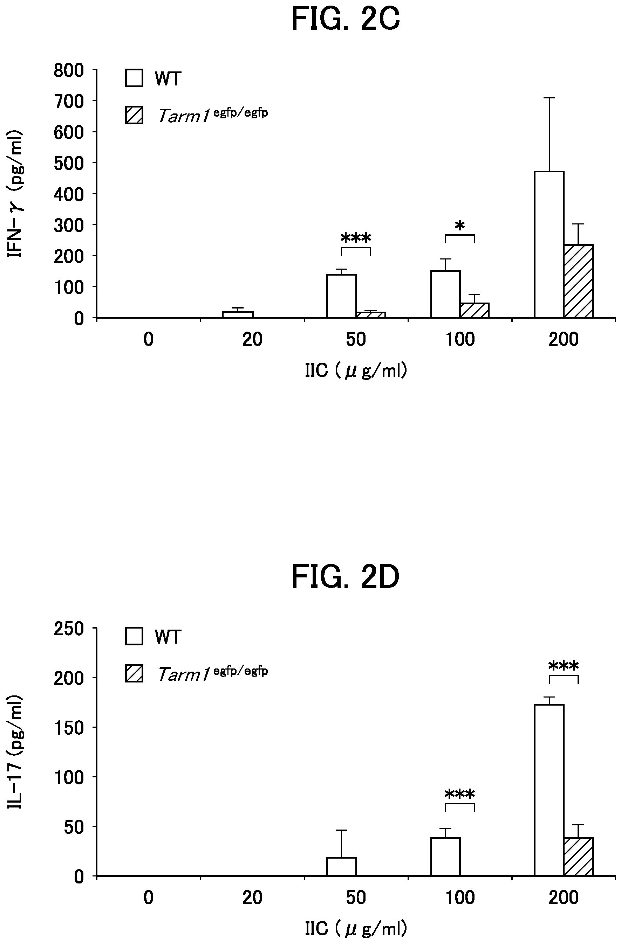

Firstly, the *Tarm1*^{egfp/egfp} mice and the WT mice on day 10 after CIA induction were verified for proliferating recall ability of the draining lymph nodes (dLNs). On day 10 after CIA induction, the lymph nodes were collected from the *Tarm1*^{egfp/egfp} mice and the WT mice, re-stimulated with type II collagen at various concentrations in vitro for 66 hours, and then measured for [³H]-thymidine uptake into an acid precipitable fraction. The results are shown in Fig. 2A. As shown in Fig. 2A, the proliferating recll ability for the type II collagen was decreased in dLN cells from the *Tarm1*^{egfp/egfp} mice compared to dLN cells from the WT mice.

Then, amounts of proinflammatory cytokines contained in culture supernatants after the dLN cells were re-stimulated with the type II collagen at various concentrations in vitro for 66 hours were measured with ELISA. Figs. 2B to 2D shows the amounts of the proinflammatory cytokines contained in the culture supernatants (TNF, INF-γ, and IL-17). As shown in Figs. 2B to 2D, the amounts of the proinflammatory cytokines contained in the culture supernatant of the dLN cells from the *Tarm1*^{egfp/egfp} mice were significantly lower compared to the amounts of the proinflammatory cytokines contained in the culture supernatant of the dLN cells from the WT mice.

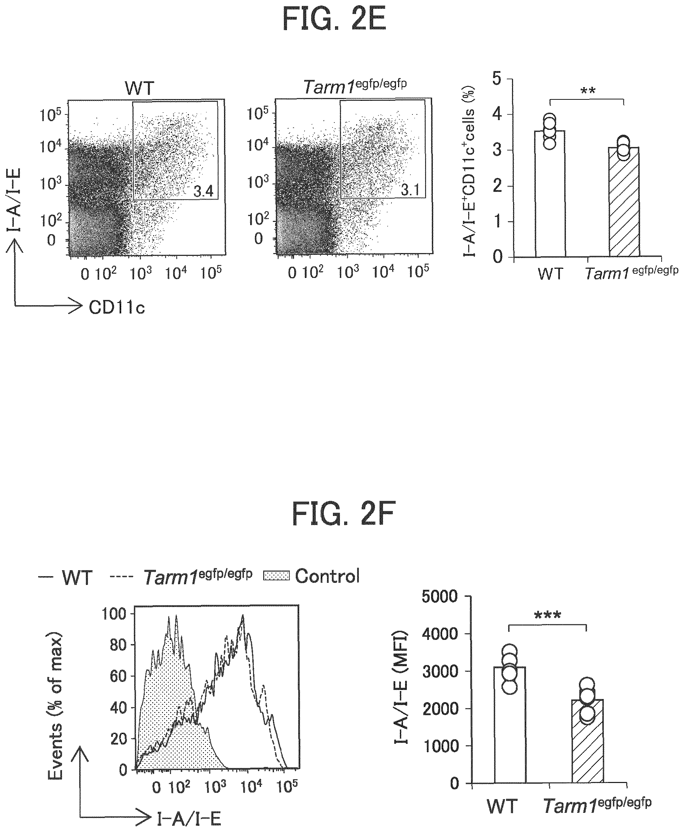

Then, the *Tarm1*^{egfp/egfp} mice and the WT mice on day 10 after CIA induction were verified for activation of dendritic cells in the draining lymph nodes (dLNs). Fig. 2E shows results of flow cytometry analysis of a I-A/I-E⁺CD11c⁺ cell population in the draining lymph nodes (dLNs) (*Tarm1*^{egfp/egfp} mouse; n = 6, WT mouse; n = 6). As shown in Fig. 2E, the I-A/I-E⁺CD11c⁺ cell population was significantly decreased in the *Tarm1*^{egfp/egfp} mice compared to the WT mice.

Then, the *Tarm1*^{egfp/egfp} mice and the WT mice on day 10 after CIA induction were verified for expression of I-A/I-E in CD11c⁺ cells in the regional lymph nodes (dLNs). Fig. 2F shows results of flow cytometry analysis of the expression of I-A/I-E in the CD11c⁺ cells in the draining lymph nodes (dLNs) (*Tarm1*^{egfp/egfp} mice; n = 6, WT mice; n = 6). As shown in Fig. 2F, the expression of I-A/I-E was significantly decreased in the CD11c⁺ cells from the *Tarm1*^{egfp/egfp} mice compared to the CD11c⁺ cells from the WT mice.

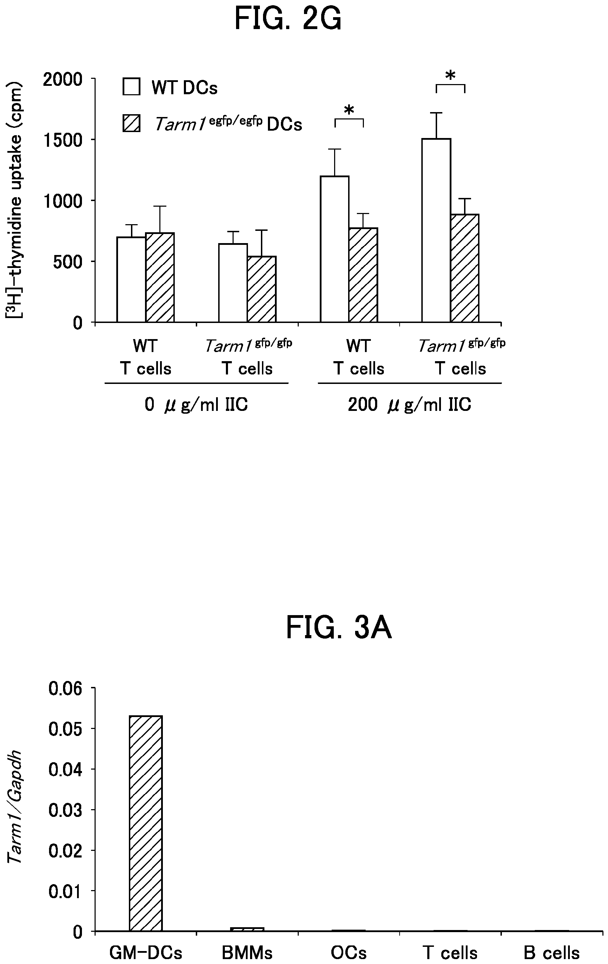

Then, a priming capability of dendritic cells for type II collagen was verified when dendritic cells and T cells isolated from the *Tarm1*^{egfp/egfp} mice and the WT mice were co-cultured. On day 10 after CIA induction, the dendritic cells and the T cells were isolated from the draining lymph nodes (dLNs) from the *Tarm1*^{egfp/egfp} mice and the WT mice and co-cultured in the presence of the type II collagen. Fig. 2G shows a result for T cell proliferation verified by [³H]-thymidine uptake. As shown in Fig. 2G, the T cells isolated from the *Tarm1*^{egfp/egfp} mice and the WT mice were significantly proliferated by co-culturing with the dendritic cells isolated from the WT mice. However, proliferation of the T cells was not observed when the T cells isolated from the *Tarm1*^{egfp/egfp} mice and the WT mice were co-cultured with the dendritic cells isolated from the WT mice.

It was suggested from the above results that T cell priming was attenuated due to a decreased function of the dendritic cells from the *Tarm1*^{egfp/egfp} mice_{.}

### <Test Example 3>

A function of Tarm1 in an immune system was examined. Firstly, an expression amount of the Tarm1 gene in GM-CSF-induced dendritic cells (GM-DCs), M-CSF-induced bone marrow-derived macrophages (BMMs), osteoclasts (OCs), T cells (Thy1.2⁺ cells), and B cell (B220⁺ cells) was analyzed by quantitative RT-PCR. Fig. 3A shows an expression amount of the Tarm1 gene in each cell. As shown in Fig. 3A, the Tarm1 gene was remarkably expressed in the GM-DCs, but was hardly expressed in the BMMs.

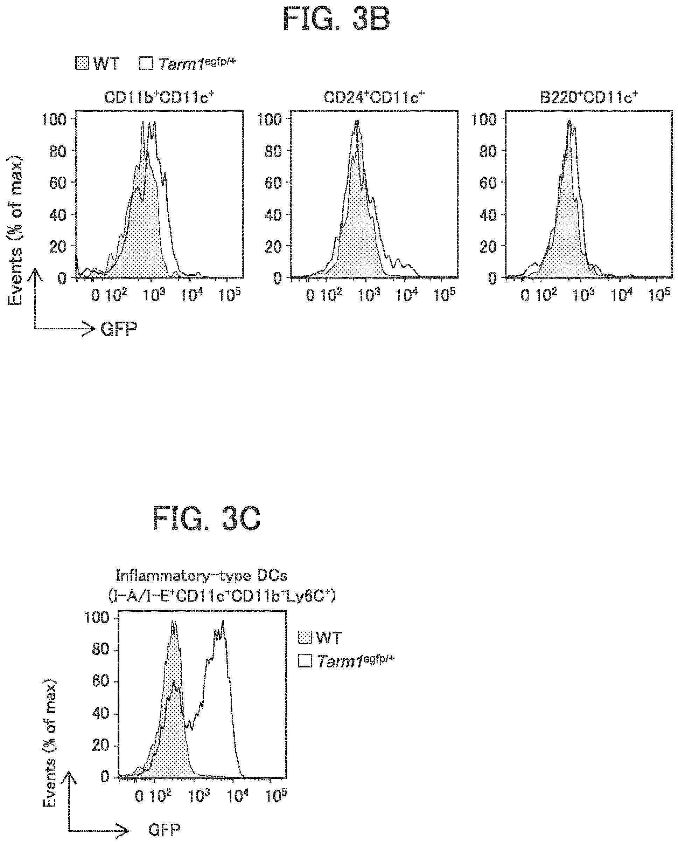

Then, an expression of the Tarm1 gene in a dendritic cell subset was verified using the *Tarm1*^{egfp/+} mice. Dendritic cells were isolated from the *Tarm1*^{egfp/+} mice and the WT mice, the expression of the Tarm1 gene was verified by analyzing an expression of an eGFP gene with flow cytometry. Fig. 3B shows results for verification of the expression of the Tarm1 gene in conventional dendritic cells (cDCs; CD11b⁺CD11c⁺ and CD24⁺CD11c⁺) and plasmacytoid dendritic cells (pDCs; B220⁺CD11c⁺). As shown in Fig. 3B, under physiological conditions, the Tarm1 gene was selectively expressed in the CD11b⁺ cDCs, but not in the CD24⁺ cDCs and the pDCs.

Then, a mixed emulsion of type II collagen and complete Freund's adjuvant (CFA) was administered to the *Tarm1*^{egfp/egfp} mice and the WT mice to thereby induce CIA, dendritic cells were isolated on day 7 after CIA induction, and the expression of the Tarm1 gene was verified by analyzing an expression of the eGFP gene with flow cytometry. Fig. 3C shows the expression of the Tarm1 gene in inflammatory dendritic cells (iDCs; Ly6C⁺Ly6G⁻MHC II^{hi}CD11b⁺CD11c⁺) . As shown in Fig. 3C, the Tarm1 gene was highly expressed in the iDCs in the draining lymph node (dLN).

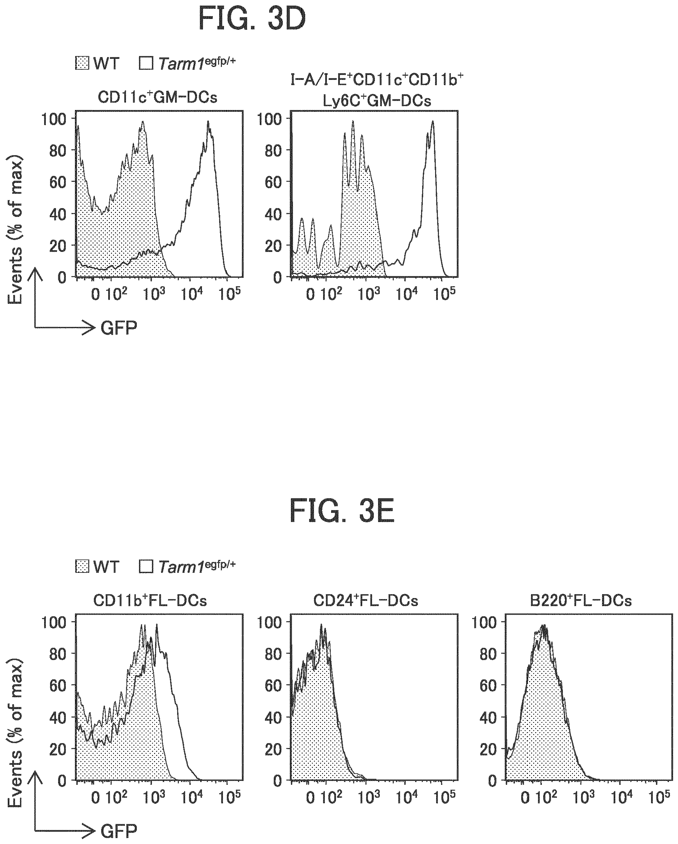

Then, an expression of the Tarm1 gene was examined in dendritic cells induced from bone marrow cells from the *Tarm1*^{egfp/+} mice and the WT mice in vitro. Fig. 3D shows results for verification of an expression of the Tarm1 gene in GM-DCs and Fig. 3E shows results for verification of an expression of the Tarm1 gene in Flt3L-induced dendritic cells (FL-DCs). As shown in Fig. 3D, the Tarm1 gene was clearly expressed in CD11b⁺ GM-DCs and more highly expressed in an inflammatory subset (I-A/I-E⁺CD11c⁺CD11b⁺Ly6C⁺) . On the other hand, as shown in Fig. 3E, the Tarm1 gene was expressed in CD11b⁺ cDCs, but not in CD24⁺ FL-DCs or B220⁺ FL-DCs.

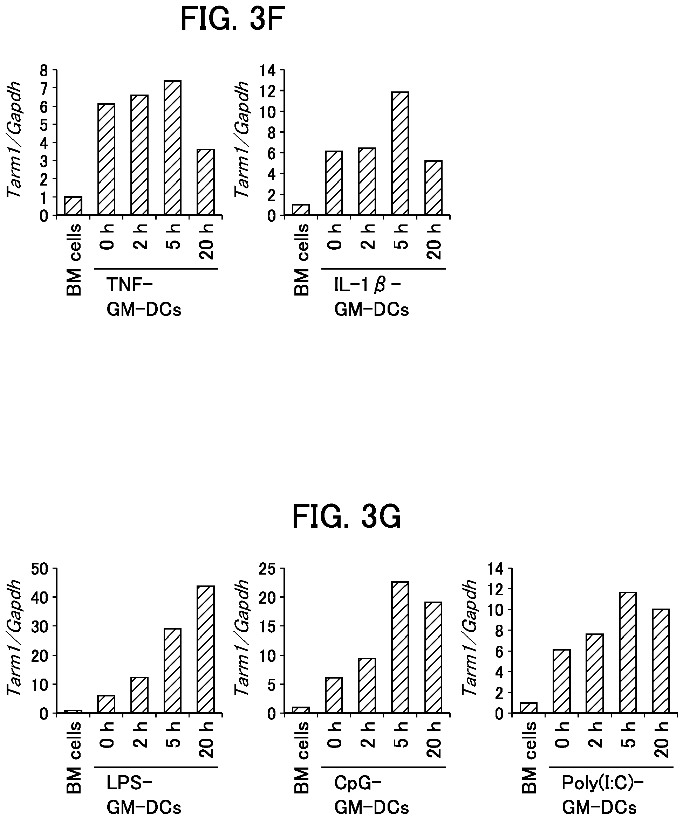

Then, whether the expression of Tarm1 gene is induced by stimulating GM-DCs was verified. GM-DCs were treated with inflammatory cytokines (TNF and IL-1β) and pathogen-associated molecular patterns (LPS, CpG, and poly(I:C)) and then an expression amount of the Tarm1 gene was analyzed with quantitative RT-PCR. Figs. 3F and 3G show expression amounts of the Tarm1 gene. The expression amounts in Figs. 3F and 3G are presented as an average value of two wells. As shown in Figs. 3F and 3G, the Tarm1 gene was induced to express in the GM-DCs both when stimulated with the inflammatory cytokines and when stimulated with the pathogen-associated molecular patterns.

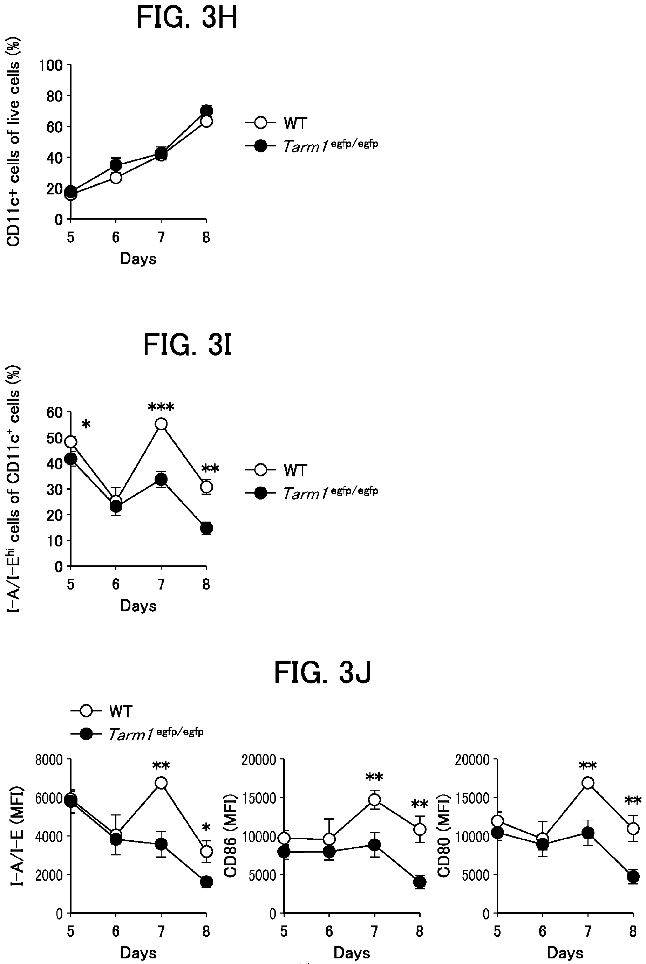

Then, percentages of CD11c⁺ cells and I-A/I-E^{hi}CD11c⁺ cells on predetermined days after a GM-CSF treatment in *Tarm1*^{egfp/egfp} mouse-derived and WT mouse-derived GM-DCs were analyzed with flow cytometry. Fig. 3H shows percentages of the CD11c⁺ cells, and Fig. 3I shows percentages of the I-A/I-E^{hi}CD11c⁺ cells (n = 3). As shown in Fig. 3H, the percentages of the CD11c⁺ cells were nearly the same between the *Tarm1*^{egfp/egfp} mouse-derived GM-DCs and the WT mouse-derived GM-DCs. However, as shown in Fig. 3I, the percentages of the I-A/I-E^{hi}CD11c⁺ cells in the *Tarm1*^{egfp/egfp} mouse-derived GM-DCs were significantly lower than that of the WT mouse-derived GM-DCs.

Then, expression of dendritic cell activation markers (I-A/I-E, CD86, and CD80) on predetermined days after a GM-CSF treatment in the *Tarm1*^{egfp/egfp} mouse-derived and WT mouse-derived GM-DCs was analyzed with flow cytometry. The results are shown in Fig. 3J (n = 3). As shown in Fig. 3J, expression of the dendritic cell activation markers was significantly decreased in the *Tarm1*^{egfp/egfp} mouse-derived GM-DCs compared to the WT mouse-derived GM-DCs.

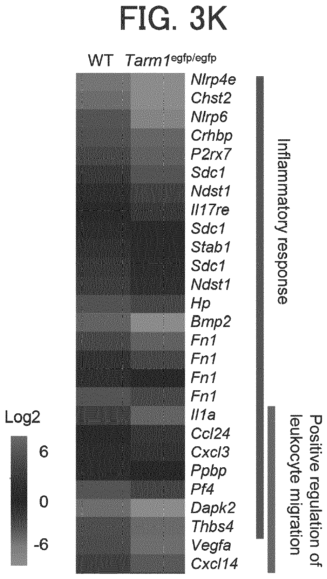

Then, expression of the genes in the *Tarm1*^{egfp/egfp} mouse-derived and WT mouse-derived GM-DCs was analyzed with a DNA microarray. Fig. 3K shows a heat map of genes of which a difference in expression amount was more than 2 times. It is revealed from the results of the gene ontology analysis that an immune function associated with an inflammatory reaction and positive regulation of leukocyte migration was decreased in the *Tarm1*^{egfp/egfp} mouse-derived GM-DCs.

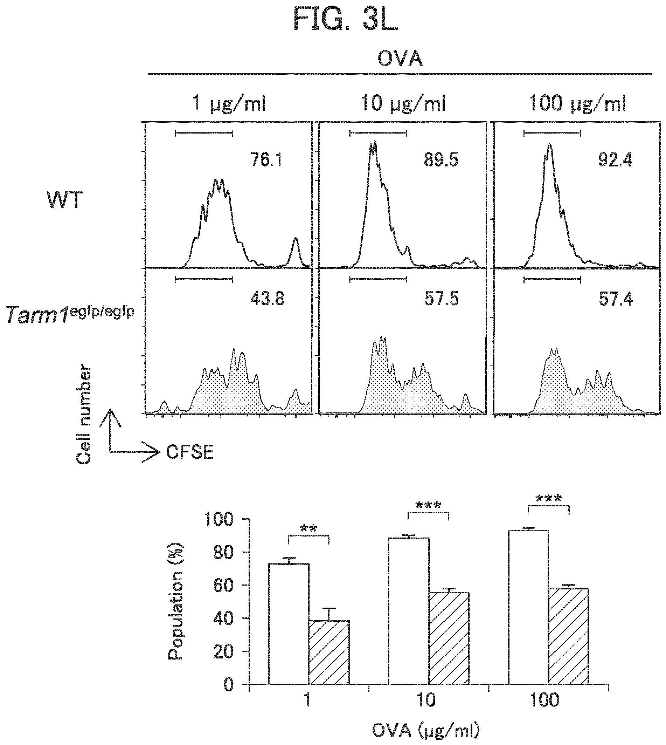

Then, an effect of deficiency in the Tarm1 gene on an antigen-presenting capability of GM-DCs was examined. OT-II Tg mouse-derived CFSE-labeled CD4⁺ T cells were co-cultured with the *Tarm1*^{egfp/egfp} mouse-derived and WT mouse-derived GM-DCs in the presence of OVA at predetermined concentrations. A CFSE intensity was measured with flow cytometry on day 3 of culture. The results are shown in Fig. 3L (n = 3). As shown in Fig. 3L, T cells were suppressed from proliferating when the OT-II Tg mouse-derived CFSE-labeled CD4⁺ T cells were co-cultured with the *Tarm1*^{egfp/egfp} mouse-derived GM-DCs compared to when co-cultured with the WT mouse-derived GM-DCs.

It was suggested from the above results that maturation of GM-DCs was suppressed in the *Tarm1*^{egfp/egfp} mice.

### <Test Example 4>

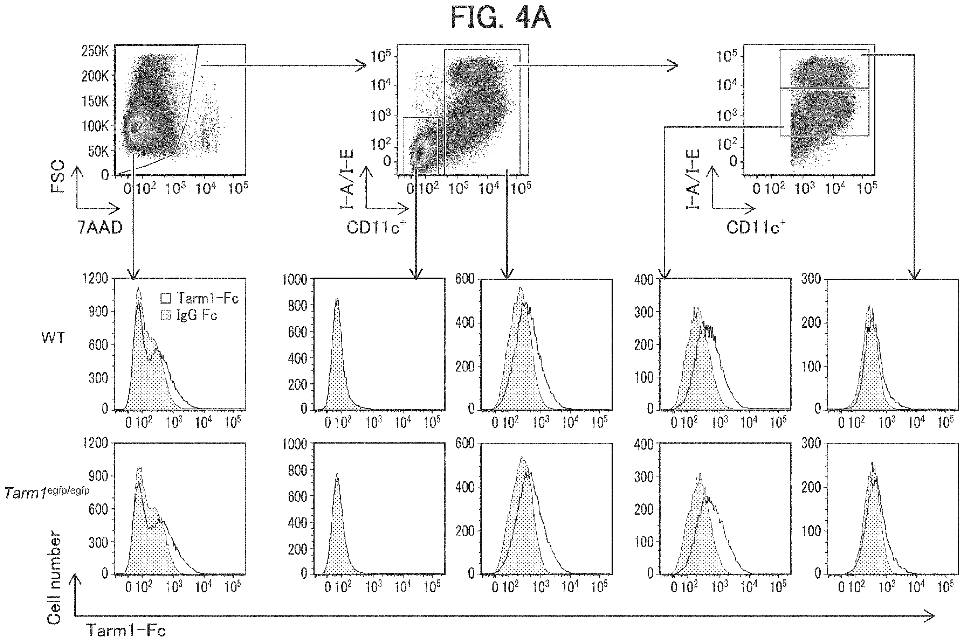

It is thought from the results of Test Examples 1 to 3 that the Tarm1 may regulate maturation of GM-DCs through selfrecognition. Therefore, ligands for the Tarm1 on the GM-DCs were analyzed with flow cytometry using Tarm1-Fc which is a fusion of an extracellular domain of the Tarm1 with an IgG Fc region. For a negative control, IgG Fc was used instead of the Tarm1-Fc. Fig. 4A shows a result for flow cytometry analysis of a binding of the *Tarm1*^{egfp/egfp} mouse-derived and WT mouse-derived GM-DCs to Tarm1-Fc. As shown in Fig. 4A, the Tarm1-Fc was bound to some of 7AAD⁻ live cells among WT mouse-derived GM-DCs. As a result of an additional analysis through costaining with an anti-CDllc antibody and an anti-I-A/I-E antibody, the Tarm1-Fc was specifically bound to CD11c⁺ cells. Furthermore, the Tarm1-Fc was bound to an I-A/I-E^{hi} group and an I-A/I-E^{int} group among the CD11c⁺ cells.

An immunoglobulin-like member is known to act in cis on itself (A. D. Barrow and J. Trowsdale, Immunological reviews, 224, 98-123, 2008; J. Trowsdale et al., Immunological reviews, 267, 117-136, 2015)_{.} Therefore, the *Tarm1*^{egfp/egfp} mouse-derived GM-DCs were also analyzed with flow cytometry for binding to the Tarm1-Fc in the same manner, and the Tarm1-Fc was found to bind to the *Tarm1*^{egfp/egfp} mouse-derived CD11c⁺ cells (Fig. 4A) .

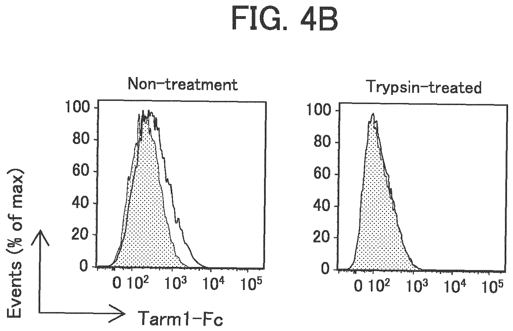

Then, a binding of trypsinized or untreated GM-DCs to Tarm1-Fc was analyzed with flow cytometry. For a negative control, IgG Fc was used instead of the Tarm1-Fc. The results are shown in Fig. 4B. As shown in Fig. 4B, the Tarm1-Fc was not bound in the case of the trypsinized GM-DCs.

It has been conventionally reported that collagen is a functional ligand for OSCAR (A. D. Barrow et al., J. Immunol., 194, 3317-3326, 2015; A. D. Barrow et al., J. Clin. Invest., 121, 3505-3516, 2011; J. Haywood et al., Proc. Natl. Acad. Sci. USA, 113, 1038-1043, 2016; L. Zhou et al., Blood, 127, 529-537, 2016). Furthermore, it has also been reported that an interaction between collagen and OSCAR is involved in activation and maturation of dendritic cells (H. S, Schultz et al., J. Immunol., 194, 3169-3179, 2015). Moreover, it has also been reported that other immunoglobulin-like leukocyte receptor complexes such as leukocyte-associated immunoglobulin-like receptor 1 (LAIR1) and glycoprotein VI (GPVI) recognize collagen-like molecules (R. J. Lebbink et al., The Journal of experimental medicine, 203, 1419-1425, 2006; R. J. Lebbink et al., Biology, 28, 202-210, 2009; Y. Miura et al., The Journal of biological chemistry, 277, 46197-46204, 2002). Therefore, the possibility that the collagen-like molecules are endogenous ligands for the Tarm1 was examined as follows.

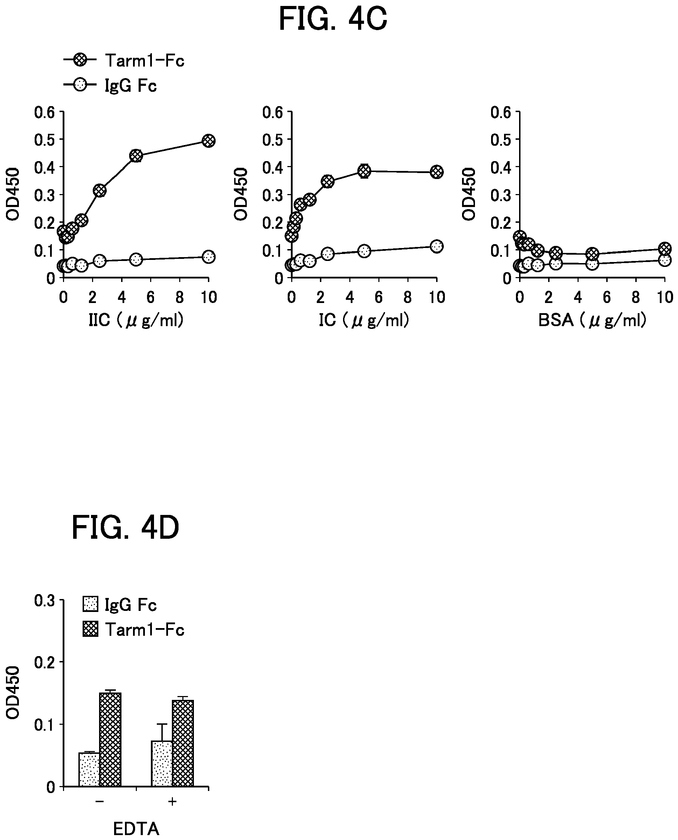

Firstly, a solid phase-binding assay was performed to examine Tarm1-Fc for its ability to bind to type I collagen, type II collagen, or BSA immobilized on plates. For a negative control, IgG Fc was used instead of the Tarm1-Fc. The results are shown in Fig. 4C. As shown in Fig. 4C, the Tarm1-Fc bound to the type I collagen and the type II collagen in a concentration-dependent manner, but did not bound to the BSA.

Furthermore, Fig. 4D shows a result for a solid phase-binding assay of a binding property of the Tarm1-Fc to the type II collagen immobilized on plates in the presence or absence of EDTA (n = 3). For a negative control, IgG Fc was used instead of the Tarm1-Fc. As shown in Fig. 4D, the Tarm1-Fc was bound to the type II collagen even in the presence of EDTA.

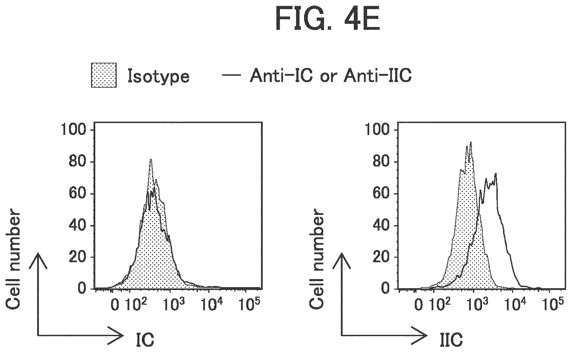

Then, the collagen-like molecules on GM-DCs were analyzed with flow cytometry. Fig. 4E shows results for flow cytometry analysis of expression of type I collagen and type II collagen on cell surfaces of GM-DCs. As shown in Fig. 4E, the type II collagen was expressed on the GM-DCs, but the type I collagen was not.

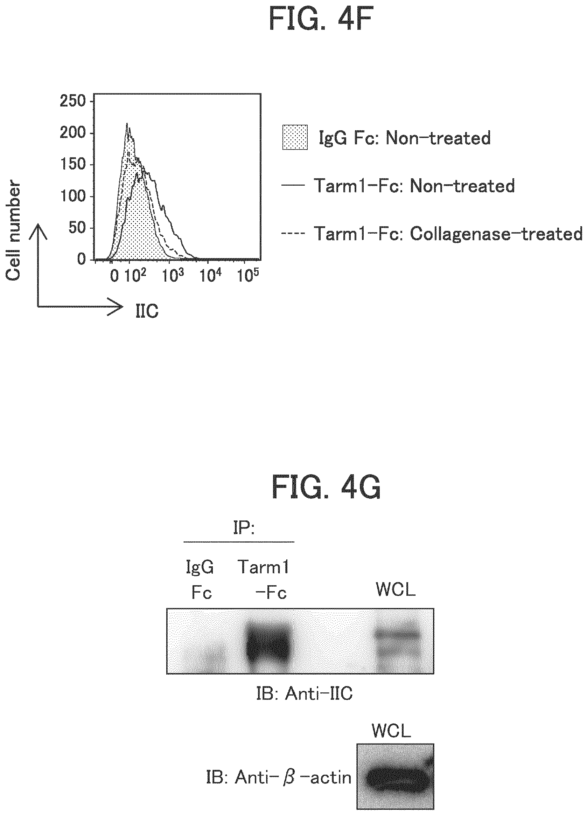

Then, a binding of collagen-treated or untreated GM-DCs to Tarm1-Fc was analyzed with flow cytometry. For a negative control, IgG Fc was used instead of the Tarm1-Fc. The results are shown in Fig. 4F. As shown in Fig. 4F, the Tarm1-Fc was not bound in the case of the collagen-treated GM-DCs.

Then, immunoprecipitation and immunoblotting (western blotting) were performed using whole cell lysates (WCLs)of GM-DCs. The whole cell lysates (WCLs) of GM-DCs were subjected to immunoprecipitation using Tarm1-Fc or IgG Fc and the resultant precipitates were subjected to immunoblotting using an anti-type II collagen antibody. Furthermore, the whole cell lysates were subjected to immunoblotting using the anti-type II collagen antibody and an anti-β-actin antibody. The results are shown in Fig. 4G. As shown in Fig. 4G, the Tarm1-Fc was bound to the type II collagen in GM-DC lysates.

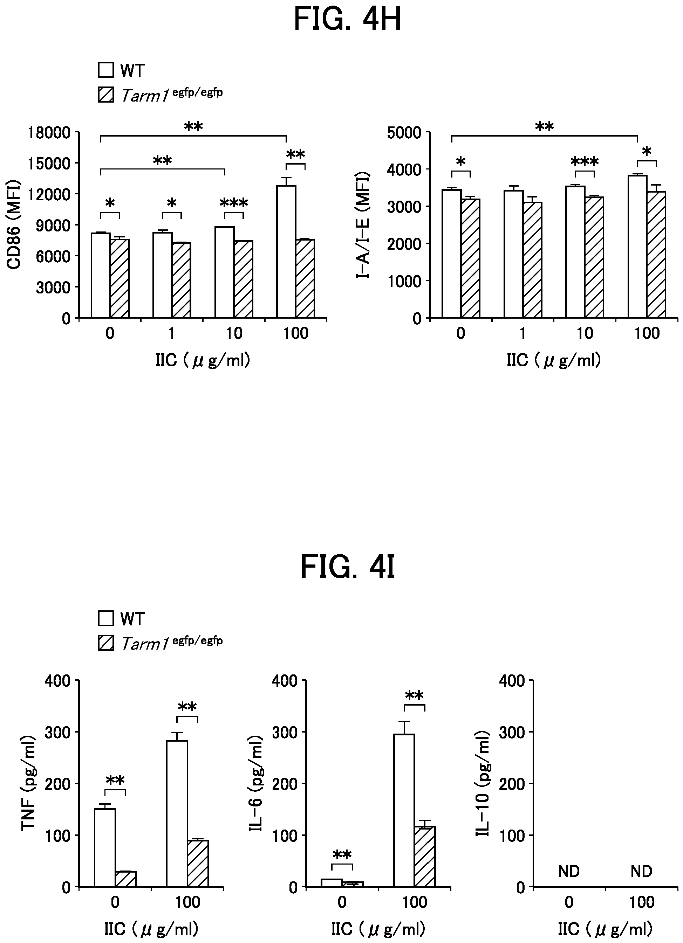

Then, the *Tarm1*^{egfp/egfp} mouse-derived and WT mouse-derived GM-DCs were stimulated with the type II collagen and expression of dendritic cell activation markers (CD86 and I-A/I-E) was analyzed with flow cytometry. The results are shown in Fig. 4H (n = 3). As shown in Fig. 4H, the expression of the dendritic cell activation markers was significantly decreased in the *Tarm1*^{egfp/egfp} mouse-derived GM-DCs compared to the WT mouse-derived GM-DCs.

Furthermore, Fig. 4I shows results for flow cytometry analysis using cytometric beads of cytokine concentrations (TNF, IL-6, and IL-10) in culture supernatants of the *Tarm1*^{egfp/egfp} mouse-derived and the WT mouse-derived GM-DCs stimulated with the type II collagen (n = 3). As shown in Fig. 4I, amounts of TNF and IL-6 in the culture supernatants were significantly decreased in the *Tarm1*^{egfp/egfp} mouse-derived GM-DCs compared to the WT mouse-derived GM-DCs.

It was suggested from the above results that the type II collagen is a ligand for the Tarm1 on the GM-DCs and regulates maturation of the GM-DCs in a Tarm1-dependent manner.

### <Test Example 5>

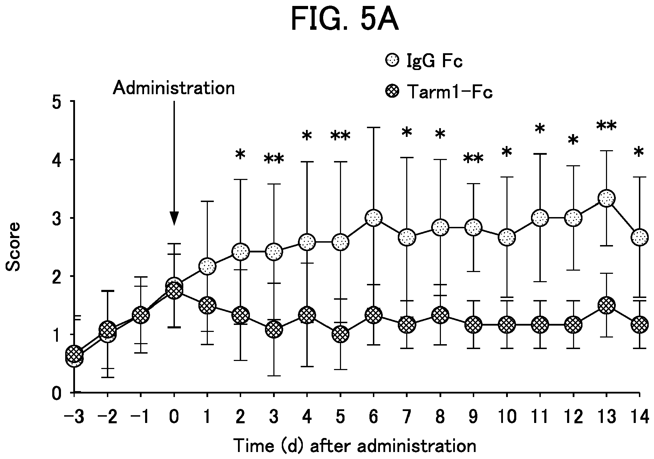

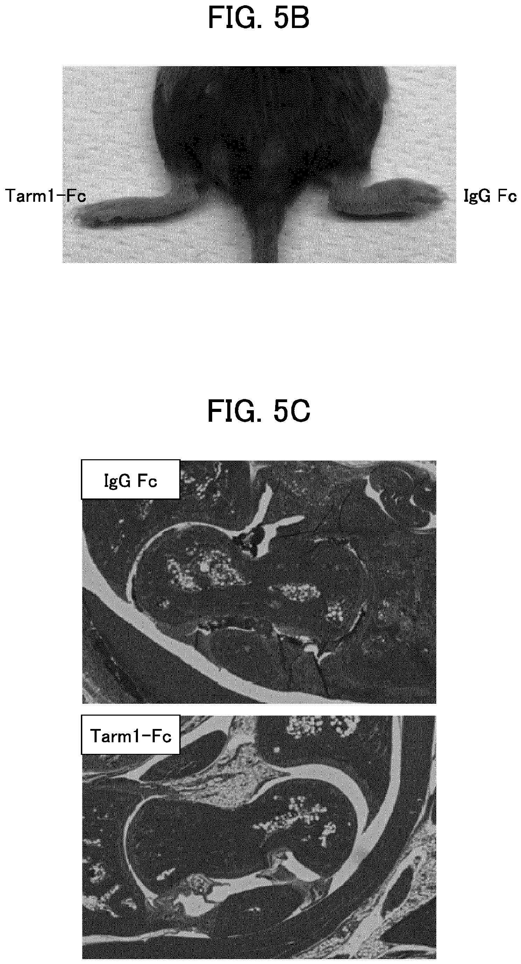

For verification of a physiological activity of the Tarm1-Fc on autoimmune arthritis, Tarm1-Fc and IgG Fc were administered to the joint cavities in the left and right ankle joints of DBA/1J mice in which CIA had been induced. A severity of arthritis is shown in Fig. 5A (n = 12). Fig. 5A shows a combination of two independent experiments. Furthermore, Fig. 5B shows joint conditions (representative examples) in the DBA/1J mice that received the Tarm1-Fc and the IgG Fc. As shown in Figs. 5A and 5B, the severity of arthritis was remarkably decreased in mice that received the Tarm1-Fc.

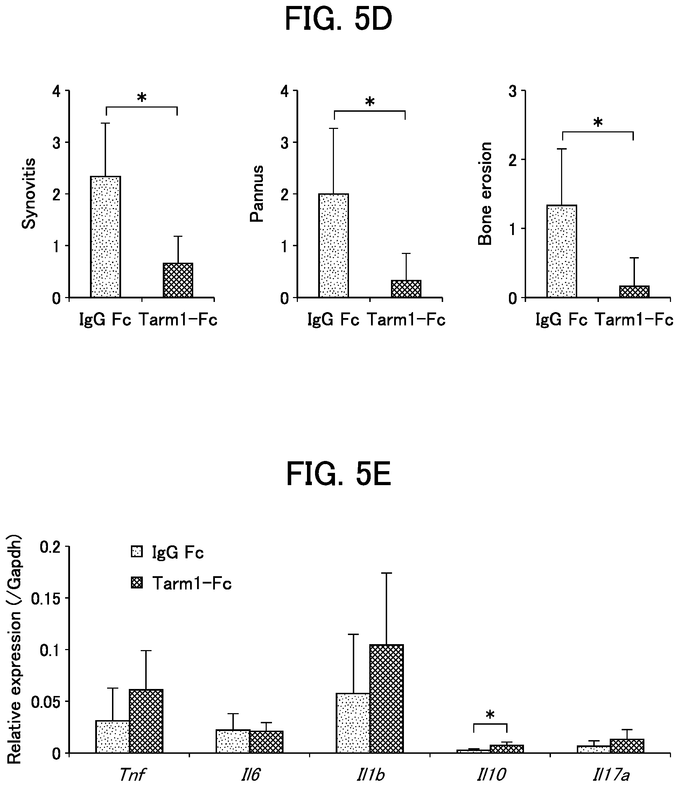

Fig. 5C illustrates images (representative examples) of hematoxylin and eosin (HE)-stained tissues in the ankle joints of the DBA/1J mice that received the Tarm1-Fc and the IgG Fc. Furthermore, Fig. 5D shows histopathological scores including synovitis, pannus formation, and bone erosion (n = 6). As shown in Figs. 5C and 5D, the synovitis, the pannus formation, and the bone erosion were remarkably decreased in the case where the Tarm1-Fc was administered.

Then, expression of cytokine (TNF, IL-6, IL-1β, IL-10, and IL-17a) genes in the joints of the DBA/1J mice that received the Tarm1-Fc and the IgG Fc was analyzed with quantitative PCR. The results are shown in Fig. 5E. As shown in Fig. 5E, an expression of the IL-10 gene was significantly enhanced in the case where the Tarm1-Fc was administered.

It was suggested from the above results that administration of the Tarm1-Fc suppress progression of autoimmune arthritis.

## Claims

1. A dendritic cell maturation-suppressing agent, the agent being capable of inhibiting binding between a TARM1 protein and a collagen protein to suppress dendritic cell maturation.

2. The dendritic cell maturation-suppressing agent according to claim 1, the agent being capable of binding to a collagen-binding domain in the TARM1 protein or the collagen protein.

3. The dendritic cell maturation-suppressing agent according to claim 2, the agent being an antibody capable of binding to the collagen-binding domain in the TARM1 protein or the collagen protein, or a functional fragment of the antibody.

4. The dendritic cell maturation-suppressing agent according to claim 2, the agent being the TARM1 protein or the collagen protein, or a functional equivalent thereof.

5. The dendritic cell maturation-suppressing agent according to claim 4, the agent being any one of polypeptides (a) to (d) below or a fusion protein of the polypeptide with an IgG Fc region:
(a) a polypeptide comprising at least amino acid residues at positions 27 to 120 or 124 to 219 from an N terminus in an amino acid sequence set forth in SEQ ID NO: 2;
(b) a polypeptide comprising at least amino acid residues at positions 27 to 120 or 124 to 218 from an N terminus in an amino acid sequence set forth in SEQ ID NO: 4;
(c) a polypeptide comprising an amino acid sequence in which one or several amino acid residues are substituted for, deleted from, or added to an amino acid sequence of the polypeptide (a) or (b) and having a binding capability for the collagen protein; and
(d) a polypeptide comprising an amino acid sequence having 90% or more sequence identity to an amino acid sequence of the polypeptide (a) or (b) and having a binding capability for the collagen protein.

6. The dendritic cell maturation-suppressing agent according to claim 4, the agent being a collagen polypeptide, a polypeptide comprising a collagen-like domain, or a fusion protein of the collagen polypeptide or the polypeptide comprising a collagen-like domain with an IgG Fc region.

7. A method for suppressing maturation of a dendritic cell, the method comprising:
bringing the dendritic cell maturation-suppressing agent according to any one of claims 1 to 6 into contact with an immature dendritic cell.

8. A pharmaceutical composition for use in treating a dendritic cell-mediated disease, comprising the dendritic cell maturation-suppressing agent according to any one of claims 1 to 6 as an active ingredient.

9. The pharmaceutical composition according to claim 8 for use in treating rheumatoid arthritis.

10. A screening method, comprising:
measuring binding activity between any one of polypeptides (a) to (d) below and a collagen protein in the presence and absence of a test substance; and
selecting the test substance as a candidate for a dendritic cell maturation-suppressing agent when the binding activity in the presence of the test substance is lower than the binding activity in the absence of the test substance:
(a) a polypeptide comprising at least amino acid residues at positions 27 to 120 or 124 to 219 from an N terminus in an amino acid sequence set forth in SEQ ID NO: 2;
(b) a polypeptide comprising at least amino acid residues at positions 27 to 120 or 124 to 218 from an N terminus in an amino acid sequence set forth in SEQ ID NO: 4;
(c) a polypeptide comprising an amino acid sequence in which one or several amino acid residues are substituted for, deleted from, or added to an amino acid sequence of the polypeptide (a) or (b) and having a binding capability for the collagen protein; and
(d) a polypeptide comprising an amino acid sequence having 90% or more sequence identity to an amino acid sequence of the polypeptide (a) or (b) and having a binding capability for the collagen protein.

11. A screening method, comprising:
measuring binding activity between any one of polypeptides (a) to (d) below and a collagen protein in the presence and absence of a test substance; and
selecting the test substance as a candidate for a therapeutic ingredient for a dendritic cell-mediated disease when the binding activity in the presence of the test substance is lower than the binding activity in the absence of the test substance:
(a) a polypeptide comprising at least amino acid residues at positions 27 to 120 or 124 to 219 from an N terminus in an amino acid sequence set forth in SEQ ID NO: 2;
(b) a polypeptide comprising at least amino acid residues at positions 27 to 120 or 124 to 218 from an N terminus in an amino acid sequence set forth in SEQ ID NO: 4;
(c) a polypeptide comprising an amino acid sequence in which one or several amino acid residues are substituted for, deleted from, or added to an amino acid sequence of the polypeptide (a) or (b) and having a binding capability for the collagen protein; and
(d) a polypeptide comprising an amino acid sequence having 90% or more sequence identity to an amino acid sequence of the polypeptide (a) or (b) and having a binding capability for the collagen protein.


SEQUENCE LISTING

<110>  TOKYO UNIVERSITY OF SCIENCE FOUNDATION

<120>  Maturation inhibitor of dendritic cells, method for inhibiting
maturation of dendritic cells and medicinal composition

<130>  RKF-058PCT

<150>  JP 2018-194614

<151>  2018-10-15

<160>  16

<210>  1
<211>  816
<212>  DNA
<213>  Homo sapiens

<220>
<221>  CDS
<222>  (1)..(816)
<223>

<400>  1
atg atc cct aag ctg ctt tcc ctc ctc tgt ttc aga ctg tgc gtg ggc       48
Met Ile Pro Lys Leu Leu Ser Leu Leu Cys Phe Arg Leu Cys Val Gly
1               5                   10                  15

caa gga gac aca agg gga gat ggg tca ctg ccc aag ccg tcc ctc agt       96
Gln Gly Asp Thr Arg Gly Asp Gly Ser Leu Pro Lys Pro Ser Leu Ser
            20                  25                  30

gcc tgg ccc agc tcg gtg gtc cct gcc aac agc aat gtg acg ctg cga      144
Ala Trp Pro Ser Ser Val Val Pro Ala Asn Ser Asn Val Thr Leu Arg
        35                  40                  45

tgt tgg act cct gcc aga ggt gtg agc ttt gtt ctc agg aag gga gga      192
Cys Trp Thr Pro Ala Arg Gly Val Ser Phe Val Leu Arg Lys Gly Gly
    50                  55                  60

att att ctg gag tcc ccg aag ccc ctt gat tct aca gag ggc gcg gcc      240
Ile Ile Leu Glu Ser Pro Lys Pro Leu Asp Ser Thr Glu Gly Ala Ala
65                  70                  75                  80

gaa ttt cac ctc aat aat cta aaa gtc aga aat gct gga gag tac acc      288
Glu Phe His Leu Asn Asn Leu Lys Val Arg Asn Ala Gly Glu Tyr Thr
                85                  90                  95

tgt gaa tac tac aga aaa gca tcc ccc cac atc ctt tca cag cgc agt      336
Cys Glu Tyr Tyr Arg Lys Ala Ser Pro His Ile Leu Ser Gln Arg Ser
            100                 105                 110

gac gtc ctt cta ctg ttg gtg aca gga cat tta tct aaa cct ttc ctc      384
Asp Val Leu Leu Leu Leu Val Thr Gly His Leu Ser Lys Pro Phe Leu
        115                 120                 125

cga acc tac caa agg ggt aca gtg acc gca ggt gga agg gtg act ctg      432
Arg Thr Tyr Gln Arg Gly Thr Val Thr Ala Gly Gly Arg Val Thr Leu
    130                 135                 140

cag tgc cag aag cga gac caa ttg ttt gtg cct atc atg ttc gct cta      480

Gln Cys Gln Lys Arg Asp Gln Leu Phe Val Pro Ile Met Phe Ala Leu
145                 150                 155                 160

ctg aag gca ggg acg cca tca ccc atc cag ctg cag agt cca gcg ggg       528
Leu Lys Ala Gly Thr Pro Ser Pro Ile Gln Leu Gln Ser Pro Ala Gly
                165                 170                 175

aag gag ata gac ttc tct ctg gtg gac gtg aca gcc ggc gat gct ggg       576
Lys Glu Ile Asp Phe Ser Leu Val Asp Val Thr Ala Gly Asp Ala Gly
            180                 185                 190

aac tac agc tgc atg tac tac cag aca aag tct ccc ttc tgg gcc tca       624
Asn Tyr Ser Cys Met Tyr Tyr Gln Thr Lys Ser Pro Phe Trp Ala Ser
        195                 200                 205

gaa ccc agt gat cag ctt gag ata ttg gtg aca gtt ccc cca ggt acc       672
Glu Pro Ser Asp Gln Leu Glu Ile Leu Val Thr Val Pro Pro Gly Thr
    210                 215                 220

aca tcg agc aac tac tcc ctg ggt aac ttc gta cga ctg ggt ctg gct       720
Thr Ser Ser Asn Tyr Ser Leu Gly Asn Phe Val Arg Leu Gly Leu Ala
225                 230                 235                 240

gcc gta att gtg gtt atc atg gga gct ttc ctg gtg gag gcc tgg tac       768
Ala Val Ile Val Val Ile Met Gly Ala Phe Leu Val Glu Ala Trp Tyr
                245                 250                 255

agc cgg aat gtg tct cca ggt gaa tca gag gcc ttc aaa cca gag tga       816
Ser Arg Asn Val Ser Pro Gly Glu Ser Glu Ala Phe Lys Pro Glu
            260                 265                 270

<210>  2
<211>  271
<212>  PRT
<213>  Homo sapiens

<400>  2

Met Ile Pro Lys Leu Leu Ser Leu Leu Cys Phe Arg Leu Cys Val Gly
1               5                   10                  15

Gln Gly Asp Thr Arg Gly Asp Gly Ser Leu Pro Lys Pro Ser Leu Ser
            20                  25                  30

Ala Trp Pro Ser Ser Val Val Pro Ala Asn Ser Asn Val Thr Leu Arg
        35                  40                  45

Cys Trp Thr Pro Ala Arg Gly Val Ser Phe Val Leu Arg Lys Gly Gly
    50                  55                  60

Ile Ile Leu Glu Ser Pro Lys Pro Leu Asp Ser Thr Glu Gly Ala Ala
65                  70                  75                  80

Glu Phe His Leu Asn Asn Leu Lys Val Arg Asn Ala Gly Glu Tyr Thr
                85                  90                  95

Cys Glu Tyr Tyr Arg Lys Ala Ser Pro His Ile Leu Ser Gln Arg Ser
            100                 105                 110

Asp Val Leu Leu Leu Leu Val Thr Gly His Leu Ser Lys Pro Phe Leu
        115                 120                 125

Arg Thr Tyr Gln Arg Gly Thr Val Thr Ala Gly Gly Arg Val Thr Leu
    130                 135                 140

Gln Cys Gln Lys Arg Asp Gln Leu Phe Val Pro Ile Met Phe Ala Leu
145                 150                 155                 160

Leu Lys Ala Gly Thr Pro Ser Pro Ile Gln Leu Gln Ser Pro Ala Gly
                165                 170                 175

Lys Glu Ile Asp Phe Ser Leu Val Asp Val Thr Ala Gly Asp Ala Gly
            180                 185                 190

Asn Tyr Ser Cys Met Tyr Tyr Gln Thr Lys Ser Pro Phe Trp Ala Ser
        195                 200                 205

Glu Pro Ser Asp Gln Leu Glu Ile Leu Val Thr Val Pro Pro Gly Thr
    210                 215                 220

Thr Ser Ser Asn Tyr Ser Leu Gly Asn Phe Val Arg Leu Gly Leu Ala
225                 230                 235                 240

Ala Val Ile Val Val Ile Met Gly Ala Phe Leu Val Glu Ala Trp Tyr
                245                 250                 255

Ser Arg Asn Val Ser Pro Gly Glu Ser Glu Ala Phe Lys Pro Glu
            260                 265                 270

<210>  3
<211>  925
<212>  DNA
<213>  Mus musculus

<220>
<221>  CDS
<222>  (49)..(915)
<223>

<400>  3
gctgatagta gacctgctga agacctttgg accagccgct gagccacc atg atc tct       57
                                                     Met Ile Ser
                                                     1

agg ctc ctt tcc ctt ctc tgc ctc cgg ctg tgt gtt ggg caa aca gac       105
Arg Leu Leu Ser Leu Leu Cys Leu Arg Leu Cys Val Gly Gln Thr Asp
    5                   10                  15

att cct gaa aat ggg tct cct ccc aag ccc agc ctc agt gcc tgg ccc 153
Ile Pro Glu Asn Gly Ser Pro Pro Lys Pro Ser Leu Ser Ala Trp Pro
20                  25                  30                  35

agc aca gtg ctt ccc acc aag agc cac gtg aca atg caa tgt aag agc 201
Ser Thr Val Leu Pro Thr Lys Ser His Val Thr Met Gln Cys Lys Ser
                40                  45                  50

ccc acc ccg agt aaa tac ttc atc ctc aaa aag gaa ggt ttc gct ttg 249
Pro Thr Pro Ser Lys Tyr Phe Ile Leu Lys Lys Glu Gly Phe Ala Leu
            55                  60                  65

aat tct gtg aag cca tat aat ttg aca gag gag acg gct gat ttt cat 297
Asn Ser Val Lys Pro Tyr Asn Leu Thr Glu Glu Thr Ala Asp Phe His
        70                  75                  80

ttc acc gac cta cga cag aat gat ggc gga cac tac acc tgt gaa tac 345
Phe Thr Asp Leu Arg Gln Asn Asp Gly Gly His Tyr Thr Cys Glu Tyr
    85                  90                  95

tat agc aaa tgg ccc cat gac aca ccg tca cac ccc agc aat gcc ctt 393
Tyr Ser Lys Trp Pro His Asp Thr Pro Ser His Pro Ser Asn Ala Leu
100                 105                 110                 115

ttc ttg ttg gtc aca ggg tac tta cct cag ccc tcc ttt caa gcc cac 441
Phe Leu Leu Val Thr Gly Tyr Leu Pro Gln Pro Ser Phe Gln Ala His
                120                 125                 130

cac cgg ggt aca gtg act gca gga agc aag gtg act ttg cag tgc cag 489
His Arg Gly Thr Val Thr Ala Gly Ser Lys Val Thr Leu Gln Cys Gln
            135                 140                 145

aaa gca ggc agt gtc ctc gga ccc gta aag ttt gcg tta ctg aag gtg 537
Lys Ala Gly Ser Val Leu Gly Pro Val Lys Phe Ala Leu Leu Lys Val
        150                 155                 160

gga cac tca act cct gtg cag aca agg agc tca aca gga atg gta tca 585
Gly His Ser Thr Pro Val Gln Thr Arg Ser Ser Thr Gly Met Val Ser
    165                 170                 175

gac ttc tct ctt cag aat gtg aca gcc aga gac tcg ggg gaa tac agc 633
Asp Phe Ser Leu Gln Asn Val Thr Ala Arg Asp Ser Gly Glu Tyr Ser
180                 185                 190                 195

tgt gtt tac tat cag gca aag gct ccc tat cgg gcc tca ggg ccc agc 681
Cys Val Tyr Tyr Gln Ala Lys Ala Pro Tyr Arg Ala Ser Gly Pro Ser
                200                 205                 210

aat ctc ctt gag atc tct gtg ata gac aac cat ctg cct caa gat ctt 729
Asn Leu Leu Glu Ile Ser Val Ile Asp Asn His Leu Pro Gln Asp Leu
            215                 220                 225

gct gcc tcg act ttc cca ccg caa ctg aca gca acc tca ccc aag acc 777
Ala Ala Ser Thr Phe Pro Pro Gln Leu Thr Ala Thr Ser Pro Lys Thr
        230                 235                 240

ccg ggt aca atg aca gaa ggc tac act gtg gat aat ctc atc cgg gtc 825
Pro Gly Thr Met Thr Glu Gly Tyr Thr Val Asp Asn Leu Ile Arg Val
    245                 250                 255

ggt gtg gct gct gca atc ctg cta ata gtg gga ggc ttc ctg gtt gaa 873
Gly Val Ala Ala Ala Ile Leu Leu Ile Val Gly Gly Phe Leu Val Glu
260                 265                 270                 275

gcc tgg cac agt gag cgg ctg tct cca aat aaa ccc tgg taa     915
Ala Trp His Ser Glu Arg Leu Ser Pro Asn Lys Pro Trp
                280                 285

aataactgaa     925

<210>  4
<211>  288
<212>  PRT
<213>  Mus musculus

<400>  4

Met Ile Ser Arg Leu Leu Ser Leu Leu Cys Leu Arg Leu Cys Val Gly
1               5                   10                  15

Gln Thr Asp Ile Pro Glu Asn Gly Ser Pro Pro Lys Pro Ser Leu Ser
            20                  25                  30

Ala Trp Pro Ser Thr Val Leu Pro Thr Lys Ser His Val Thr Met Gln
        35                  40                  45

Cys Lys Ser Pro Thr Pro Ser Lys Tyr Phe Ile Leu Lys Lys Glu Gly
    50                  55                  60

Phe Ala Leu Asn Ser Val Lys Pro Tyr Asn Leu Thr Glu Glu Thr Ala
65                  70                  75                  80

Asp Phe His Phe Thr Asp Leu Arg Gln Asn Asp Gly Gly His Tyr Thr
                85                  90                  95

Cys Glu Tyr Tyr Ser Lys Trp Pro His Asp Thr Pro Ser His Pro Ser
            100                 105                 110

Asn Ala Leu Phe Leu Leu Val Thr Gly Tyr Leu Pro Gln Pro Ser Phe
        115                 120                 125

Gln Ala His His Arg Gly Thr Val Thr Ala Gly Ser Lys Val Thr Leu
    130                 135                 140

Gln Cys Gln Lys Ala Gly Ser Val Leu Gly Pro Val Lys Phe Ala Leu
145                 150                 155                 160

Leu Lys Val Gly His Ser Thr Pro Val Gln Thr Arg Ser Ser Thr Gly
                165                 170                 175

Met Val Ser Asp Phe Ser Leu Gln Asn Val Thr Ala Arg Asp Ser Gly
            180                 185                 190

Glu Tyr Ser Cys Val Tyr Tyr Gln Ala Lys Ala Pro Tyr Arg Ala Ser
        195                 200                 205

Gly Pro Ser Asn Leu Leu Glu Ile Ser Val Ile Asp Asn His Leu Pro
    210                 215                 220

Gln Asp Leu Ala Ala Ser Thr Phe Pro Pro Gln Leu Thr Ala Thr Ser
225                 230                 235                 240

Pro Lys Thr Pro Gly Thr Met Thr Glu Gly Tyr Thr Val Asp Asn Leu
                245                 250                 255

Ile Arg Val Gly Val Ala Ala Ala Ile Leu Leu Ile Val Gly Gly Phe
            260                 265                 270

Leu Val Glu Ala Trp His Ser Glu Arg Leu Ser Pro Asn Lys Pro Trp
        275                 280                 285

<210>  5
<211>  20
<212>  DNA
<213>  Artificial sequence

<220>
<223>  Forward primer for amplifying Tarm1 gene

<400>  5
tctctaggct cctttccctt                                                   20

<210>  6
<211>  17
<212>  DNA
<213>  Artificial sequence

<220>
<223>  Reverse primer for amplifying Tarm1 gene

<400>  6
gtcacgtggc tcttggt                                                      17

<210>  7
<211>  20
<212>  DNA
<213>  Artificial sequence

<220>
<223>  Forward primer for amplifying Gapdh gene

<400>  7
ttcaccacca tggagaaggc                                                   20

<210>  8
<211>  20
<212>  DNA

<213>  Artificial sequence

<220>
<223>  Reverse primer for amplifying Gapdh gene

<400>  8
ggcatggact gtggtcatga                                                    20


<210>  9
<211>  20
<212>  DNA
<213>  Artificial sequence

<220>
<223>  Forward primer for amplifying Tnf gene

<400>  9
gcctccctct catcagttct                                                    20


<210>  10
<211>  20
<212>  DNA
<213>  Artificial sequence

<220>
<223>  Reverse primer for amplifying Tnf gene

<400>  10
cacttggtgg tttgctacga                                                    20


<210>  11
<211>  23
<212>  DNA
<213>  Artificial sequence

<220>
<223>  Forward primer for amplifying Il6 gene

<400>  11
gaggatacca ctcccaacag acc                                                23


<210>  12
<211>  24
<212>  DNA
<213>  Artificial sequence

<220>
<223>  Reverse primer for amplifying Il6 gene

<400>  12
aagtgcatca tcgttgttca taca                                               24


<210>  13
<211>  25
<212>  DNA
<213>  Artificial sequence

<220>

<223>  Forward primer for amplifying Il1b gene

<400>  13
caaccaacaa gtgatattct ccatg                                              25


<210>  14
<211>  21
<212>  DNA
<213>  Artificial sequence

<220>
<223>  Reverse primer for amplifying Il1b gene

<400>  14
gatccacact ctccagctgc a                                                  21


<210>  15
<211>  23
<212>  DNA
<213>  Artificial sequence

<220>
<223>  Forward primer for amplifying Il10 gene

<400>  15
gtggagcagg tgaagagtga ttt                                                23


<210>  16
<211>  23
<212>  DNA
<213>  Artificial sequence

<220>
<223>  Reverse primer for amplifying Il10 gene

<400>  16
tccctggatc agatttagag agc                                                23